# EUROPEAN PATENT SPECIFICATION

(11) **EP 1 972 257 B1**
(45) Date of publication and mention of the grant of the patent: **15.07.2015**
(21) Application number: 07706644.7
(22) Date of filing: 12.01.2007
(51) Int. Cl.: A61B 1/00, A61B 1/005

(54) **ELECTRIC BENDING ENDOSCOPE**
ELEKTRISCH BIEGENDES ENDOSKOP
ENDOSCOPE À CINTRAGE ÉLECTRIQUE

(30) Priority: 13.01.2006 JP 2006006144; 13.01.2006 JP 2006006145
(43) Date of publication of application: 24.09.2008
(73) Proprietor: Olympus Medical Systems Corp., Tokyo 151-0072 (JP)
(72) Inventor: KAWAI, Toshimasa, Tokyo 151-0072 (JP)
(74) Representative: von Hellfeld, Axel
(86) International application number: PCT/JP2007/050302
(87) International publication number: WO 2007/080953

(56) References cited:
- JP-A- 2003 245 246
- US-A1- 2004 054 258
- US-A1- 2004 073 084
- US-A1- 2004 193 014

## Description

### Technical Field

The present invention relates to an electric bending endoscope in which a bending portion electrically bends into a state corresponding to an absolute position signal by operating a bending operation instruction portion for outputting the absolute position signal.

### Background Art

In these years, an endoscope has been widely used in which an elongated insertion portion is inserted into a body cavity to observe body organs in the body cavity, and as required, a treatment instrument inserted into a therapeutic device channel is used to apply various treatment and measures.

This endoscope generally has a bending portion bending up and down, and right and left provided on the side of a distal end portion thereof, and the bending portion can be bent in a desired direction by pulling and loosening a bending wire linked to the bending portion.

The bending wire has been generally operated manually, but recently, there is also an electric bending endoscope in which the bending wire is pulled by bending power means using an electric motor or the like, as disclosed in Japanese Patent Application Laid-Open Publication No. 2003-245246 and the like.

In this electric bending endoscope, for example, an electric motor was rotated with, for example, a joystick for outputting a bending instruction signal of an absolute position, the joystick being bending operation instruction means provided in an operation portion, and rotation of the electric motor rotated a pulley to pull the bending wire linked to the pulley, thereby bending the bending portion.

The joystick instructs a bending position by tilting operation. That is, a direction to which the joystick is tilted is a desired direction in which the bending portion is bent, and a tilting angle of the joystick is a bending angle of the bending portion. When the joystick is in an erect attitude having the tilting angle of 0°, the bending portion does not bend (in a straight line). Consequently, an operator can easily understand a bending state of the bending portion in the body cavity by feeling the joystick held by the operator's fingers.

In the electric bending endoscope of such a type, one finger can easily make the bending portion bend in a desired manner, and another finger can also operate the other switches provided on the operation portion to improve operability. However, the bending wire is always exposed to a tension regardless of being in a bending state or in a non-bending state, and thus the following items are requested.
(1) It is desirable that the bending wire be prevented from extending, although the bending wire tends to extend due to the tension.
(2) It is desirable that the tension not act on the bending wire during manipulative insertion to provide a bend-free state in which the bending portion can freely bend by an external force.
(3) It is desirable that the insertion portion be pulled out in a bend-free state when a failure or a trouble occurs during insertion.

Accordingly, a clutch mechanism has been provided in which the tension acting on the bending wire can be switched between being in a drive force transmission disconnected state and in a drive force transmission recovered state, as required.

However, in the electric bending endoscope disclosed in Japanese Patent Application Laid-Open Publication No. 2003-245246 described above or the like, rotation of the bending motor is monitored by an encoder, and a bending state of the bending portion is monitored by a potentiometer, but in the drive force transmission disconnected state set by the clutch mechanism, a position of the joystick will not correspond to the bending state of the bending portion, and in case of returning to the drive force transmission recovered state by the clutch mechanism, it is necessary to manually align the position of the joystick with the potentiometer, and subsequently, to restart bending control, and because this manual position adjustment of the joystick is cumbersome, there has been a problem that clutch operation cannot be efficiently and quickly carried out.

Further, in the electric bending endoscope disclosed in Japanese Patent Application Laid-Open Publication No. 2003-245246 described above, the rotation of the bending motor was monitored by the encoder, and the bending state of the bending portion was monitored by the potentiometer, and even when the drive force from the bending motor was transmitted to the bending portion by the clutch mechanism, the bending state of the bending portion was monitored by the potentiometer.

However, because the position of the bending portion detected by the potentiometer was not necessarily accurate, the position sensed on clutch connection might include an error although particularly effective on clutch disconnection, which has presented a problem of unsuitability for appropriate bending control.

The present invention has been made under the circumstances described above, and an object thereof is to provide an electric bending endoscope allowing for easy position adjustment of a joystick corresponding to a bending state even if switching between a drive force transmission disconnected state and a drive force transmission recovered state are performed by using a clutch mechanism.

Further, another object of the present invention is to provide an electric bending endoscope in which appropriate bending drive control can be performed correspondingly to a state of a drive force transmission to a bending portion.

### Disclosure of Invention

### Means for Solving the Problem

An electric bending endoscope of the present invention includes the features of claim 1.

### Brief Description of the Drawings

Fig. 1 shows a configuration diagram of an electric bending endoscope apparatus according to a first embodiment of the present invention;
Fig. 2 shows a configuration diagram of a front panel of an image processing device in Fig. 1;
Fig. 3 shows a configuration diagram of a bending control portion in Fig. 1;
Fig. 4 shows a configuration diagram of a control portion of the bending control portion in Fig. 1;
Fig. 5 shows a configuration diagram of a logic block of an FPGA in Fig. 4;
Fig. 6 shows a configuration diagram of a control processing portion of a motor controller in Fig. 5;
Fig. 7 shows a configuration diagram of a servo error detection portion of the motor controller in Fig. 5;
Fig. 8 is a diagram illustrating servo control of the motor controller in Fig. 5;
Fig. 9 is a diagram illustrating a first variation of the servo control of the motor controller in Fig. 5;
Fig. 10 is a diagram illustrating a variation of a configuration of the FPGA in Fig. 4;
Fig. 11 is a diagram illustrating a second variation of the servo control of the motor controller in Fig. 5;
Fig. 12 is a diagram illustrating a logic element block constituting an FPGA block error monitor portion in Fig. 5;
Fig. 13 is a first diagram illustrating a logic determination block using the logic element block in Fig. 12;
Fig. 14 is a second diagram illustrating the logic determination block using the logic element block in Fig. 12;
Fig. 15 is a diagram illustrating process transition in the FPGA in Fig. 5;
Fig. 16 is a flow chart illustrating processes in the FPGA in Fig. 5;
Fig. 17 is a flow chart illustrating an initial mode process in Fig. 16;
Fig. 18 is a flow chart illustrating a maintenance mode process in Fig. 16;
Fig. 19 shows a configuration diagram of a remote control operation portion for the bending control portion on clutch connection in Fig. 3;
Fig. 20 shows a configuration diagram of the remote control operation portion for the bending control portion on clutch disconnection in Fig. 3;
Fig. 21 shows a configuration diagram of the remote control operation portion for the bending control portion on clutch reconnection in Fig. 3;
Fig. 22 is a flow chart illustrating a calibration mode process in Fig. 16;
Fig. 23 is a flow chart illustrating an alignment process in Fig. 22;
Fig. 24 is a first view illustrating the alignment process in Fig. 22;
Fig. 25 is a second view illustrating the alignment process in Fig. 22;
Fig. 26 is a third view illustrating the alignment process in Fig. 22;
Fig. 27 is a fourth view illustrating the alignment process in Fig. 22;
Fig. 28 is a fifth view illustrating the alignment process in Fig. 22;
Fig. 29 is a sixth view illustrating the alignment process in Fig. 22;
Fig. 30 is a diagram illustrating the calibration mode process in Fig. 16 in terms of signal control;
Fig. 31 shows a configuration diagram of a variation of the remote control operation portion for the bending control portion on clutch connection in Fig. 3;
Fig. 32 is a flow chart illustrating the alignment process in a configuration in Fig. 30;
Fig. 33 is a first view illustrating the calibration mode process in a configuration in Fig. 31 in terms of signal control;
Fig. 34 is a second view illustrating the calibration mode process in the configuration in Fig. 31 in terms of signal control;
Fig. 35 is a third view illustrating the calibration mode process in the configuration in Fig. 31 in terms of signal control;
Fig. 36 is a fourth view illustrating the calibration mode process in the configuration in Fig. 31 in terms of signal control;
Fig. 37 is a diagram illustrating creation of tension data substituting for means for sensing tension of an endoscope bending wire shown in Fig. 31;
Fig. 38 is a flow chart illustrating an operation mode process in Fig. 16;
Fig. 39 is a timing chart illustrating the operation mode process in Fig. 16;
Fig. 40 is a diagram illustrating position servo control of the motor controller of an FPGA according to a second embodiment of the present invention; and
Fig. 41 is a flow chart illustrating the calibration mode process in the position servo control in Fig. 40.

### Best Mode for Carrying Out the Invention

Embodiments of the present invention will be hereinafter described with reference to the accompanying drawings.

### [First Embodiment]

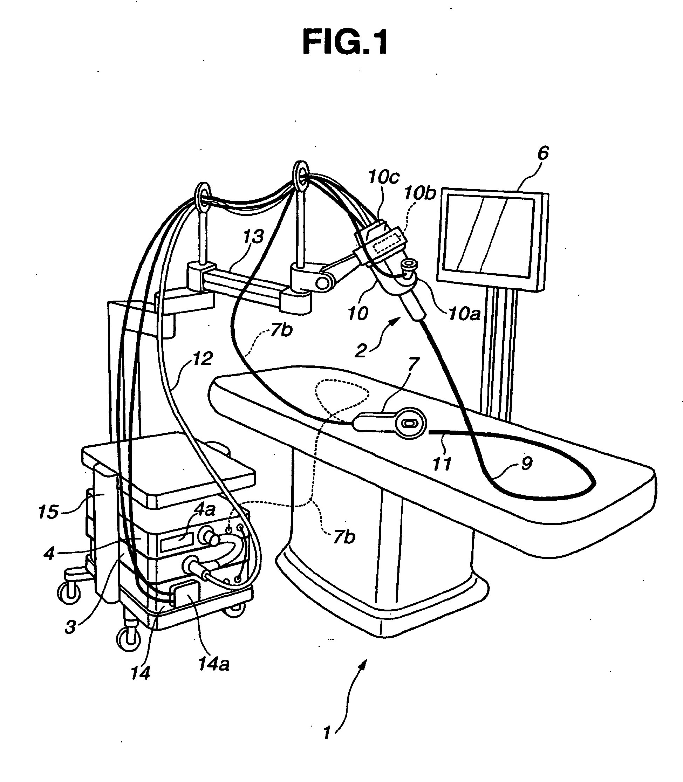

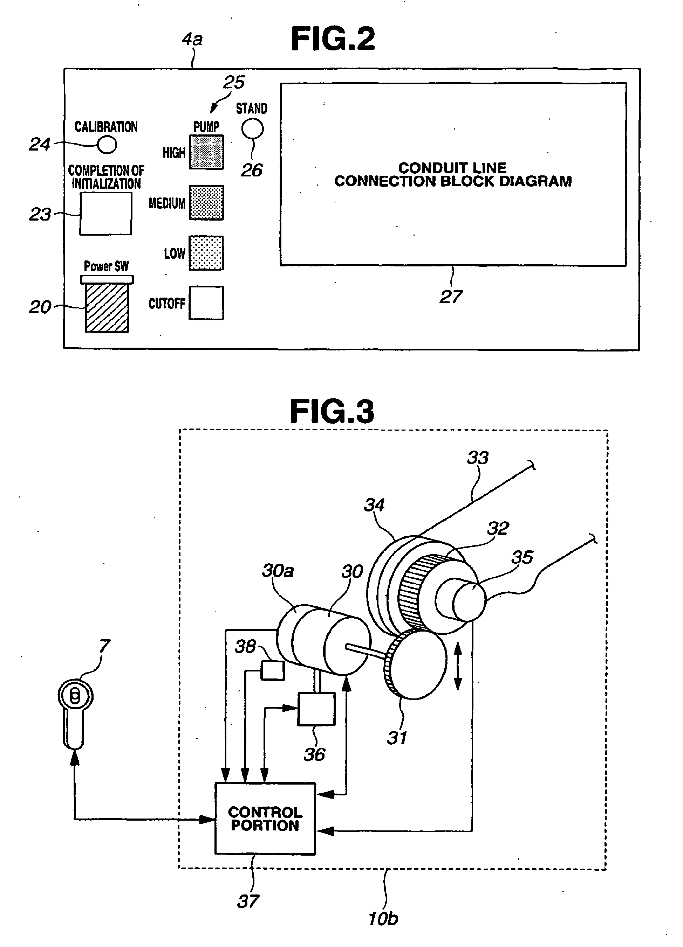

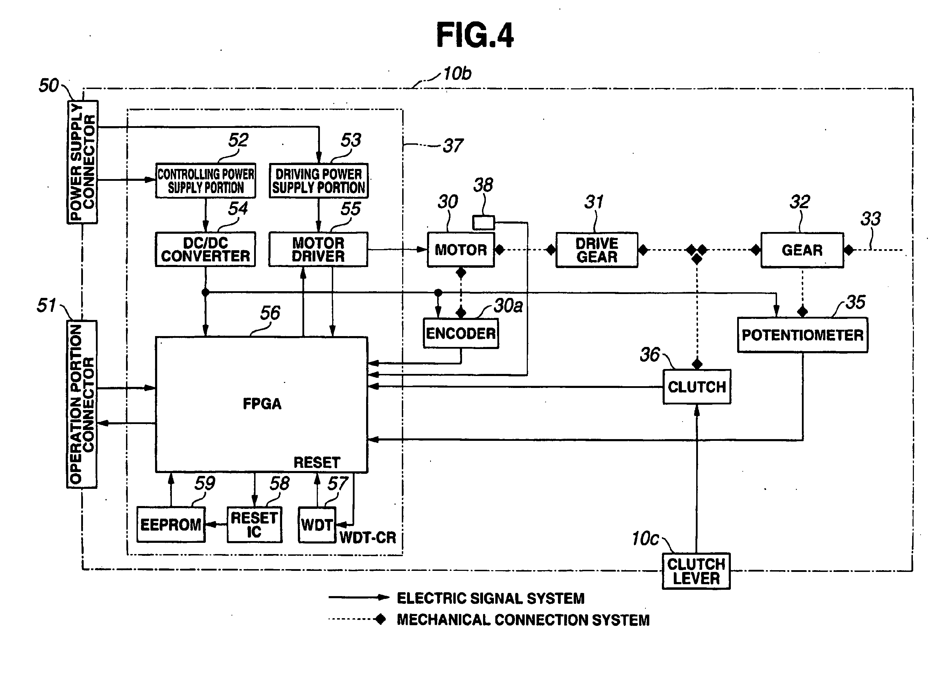

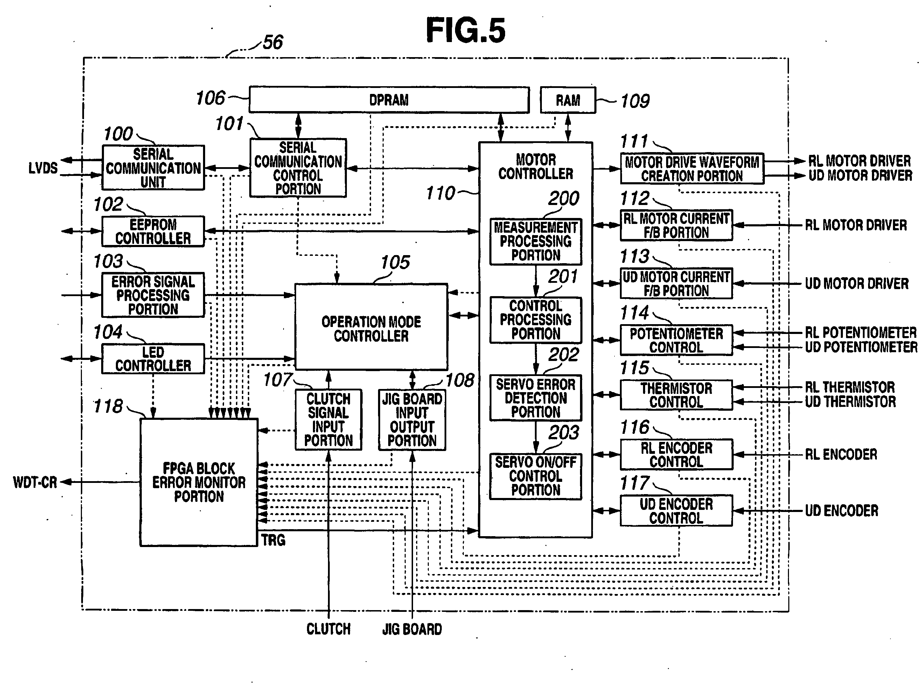

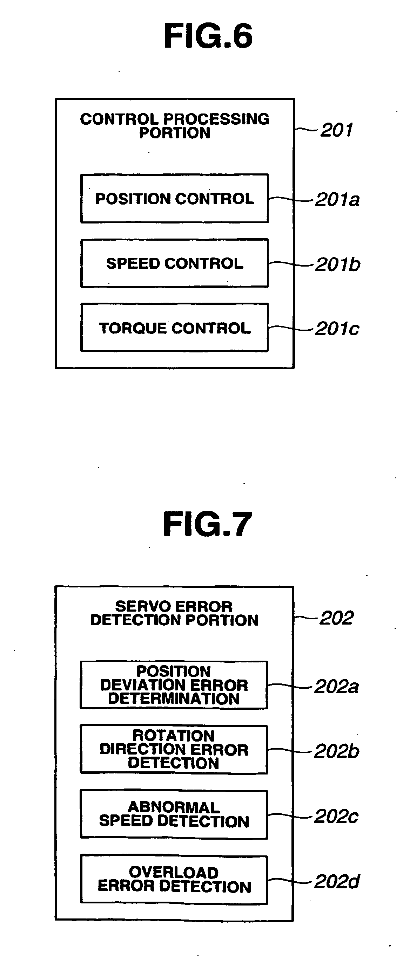

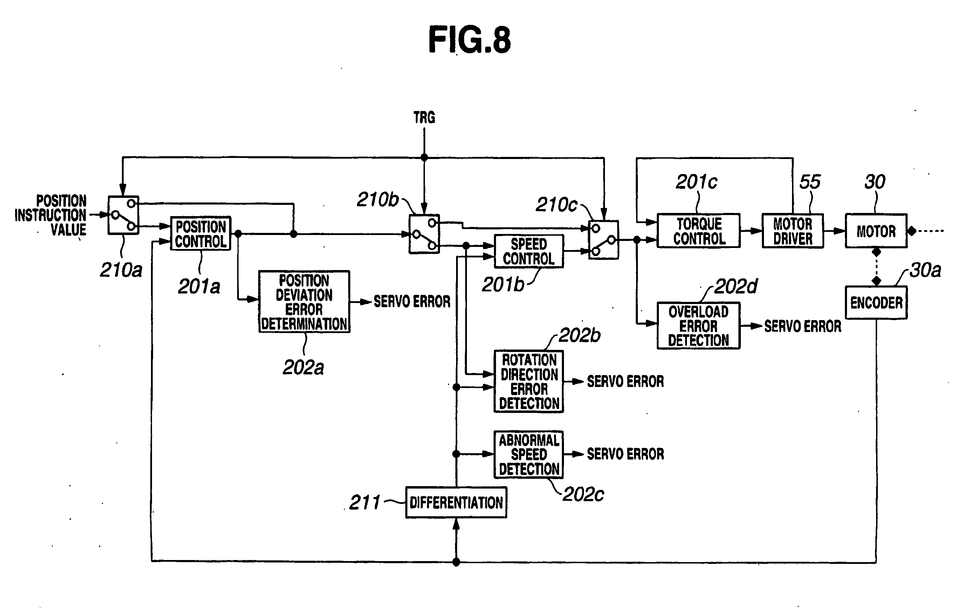

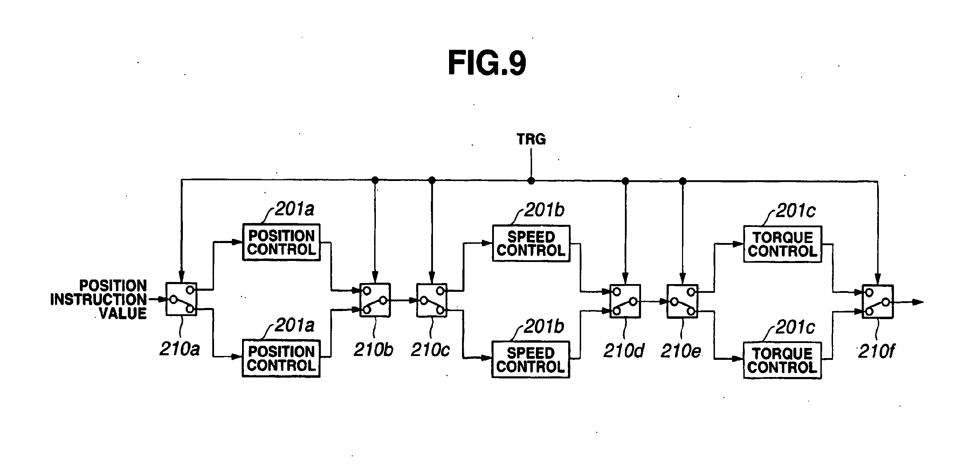

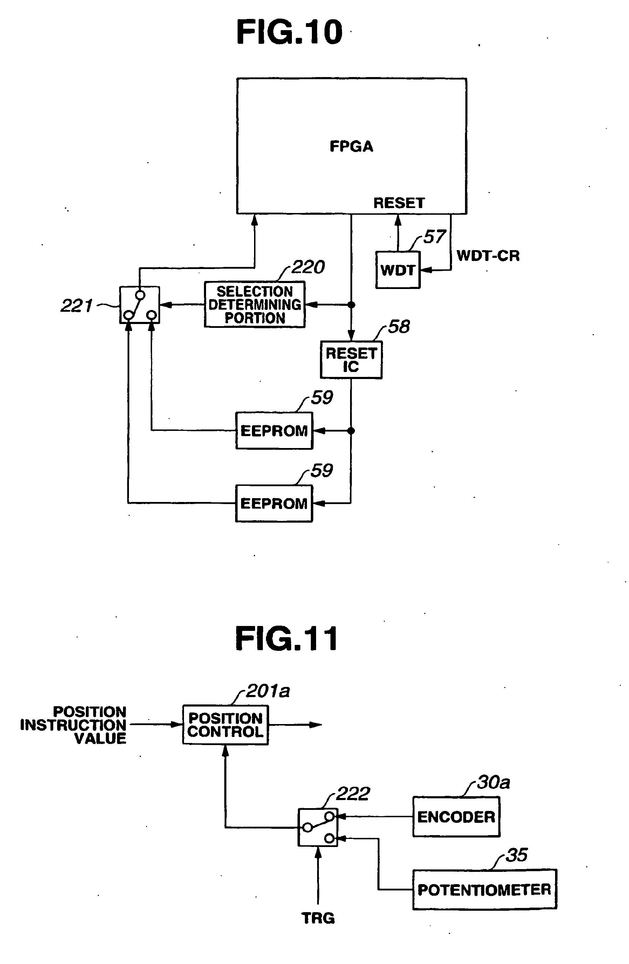

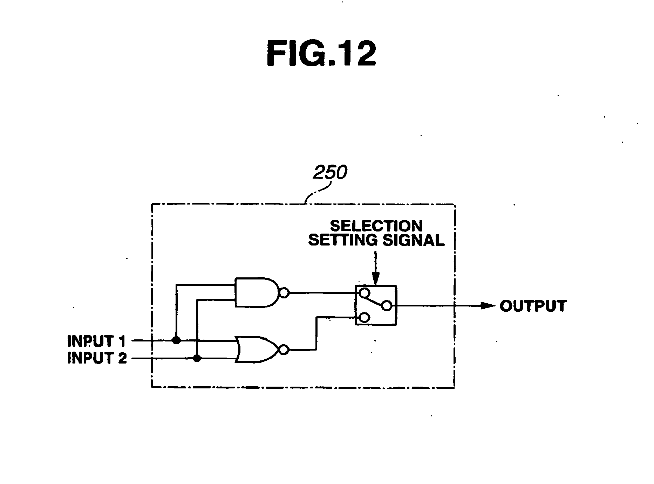

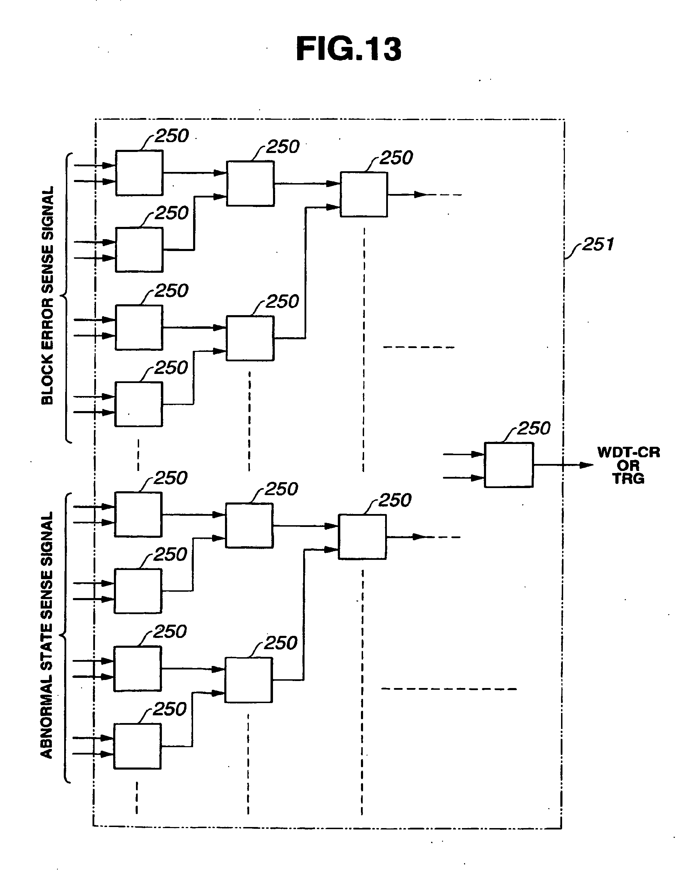

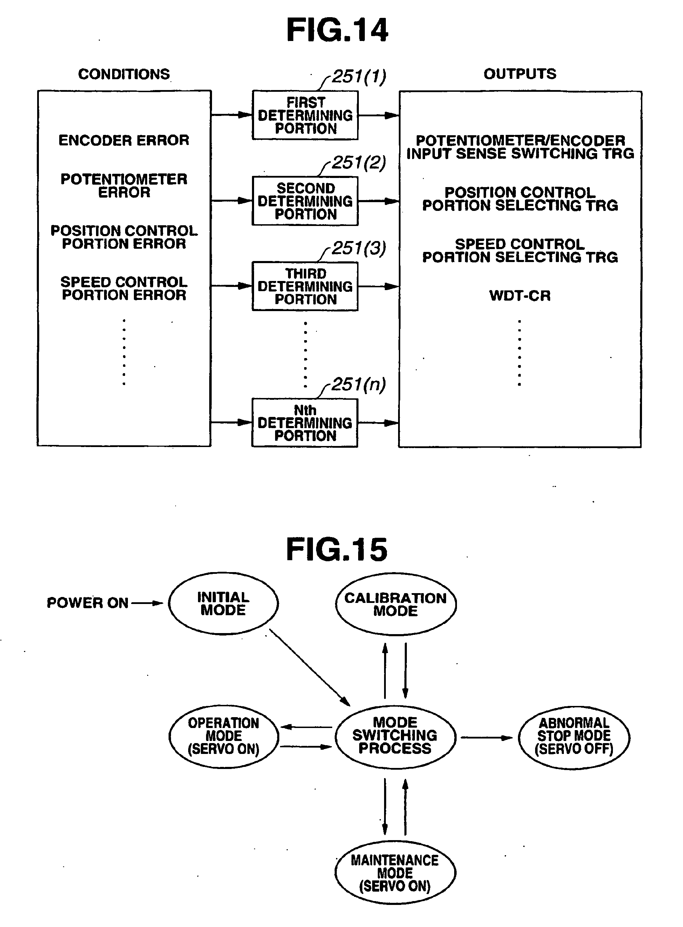

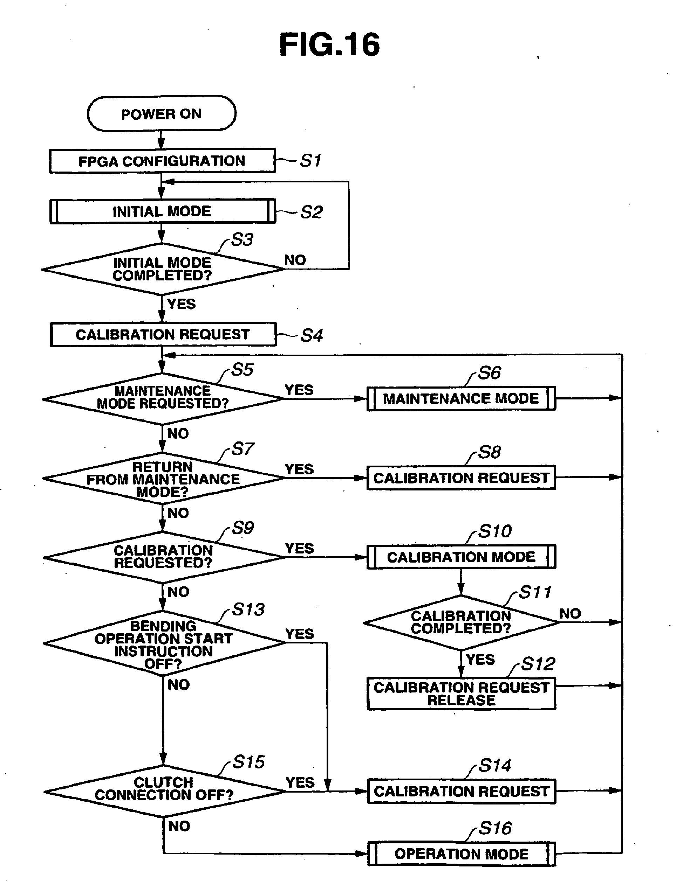

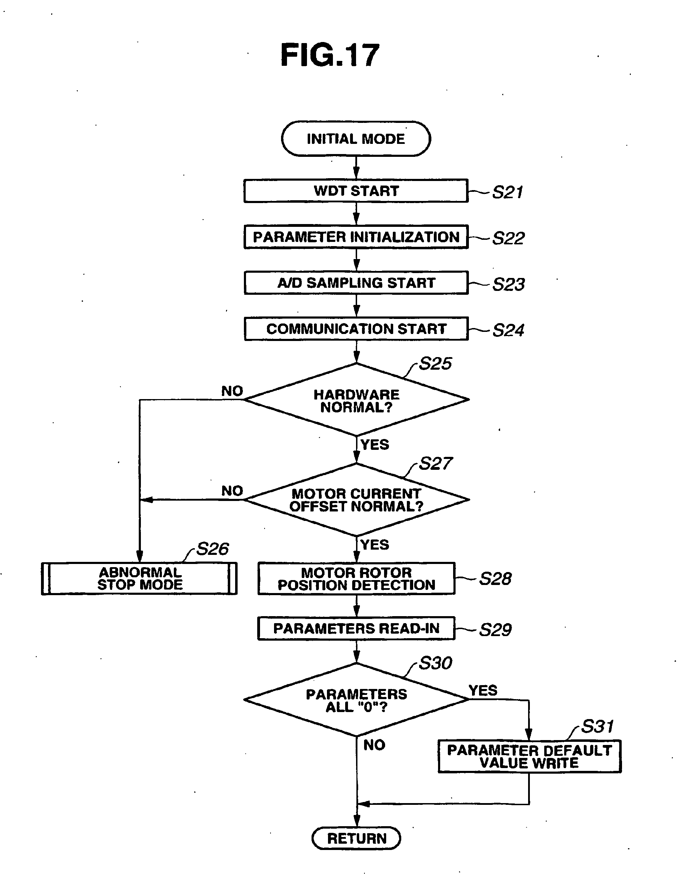

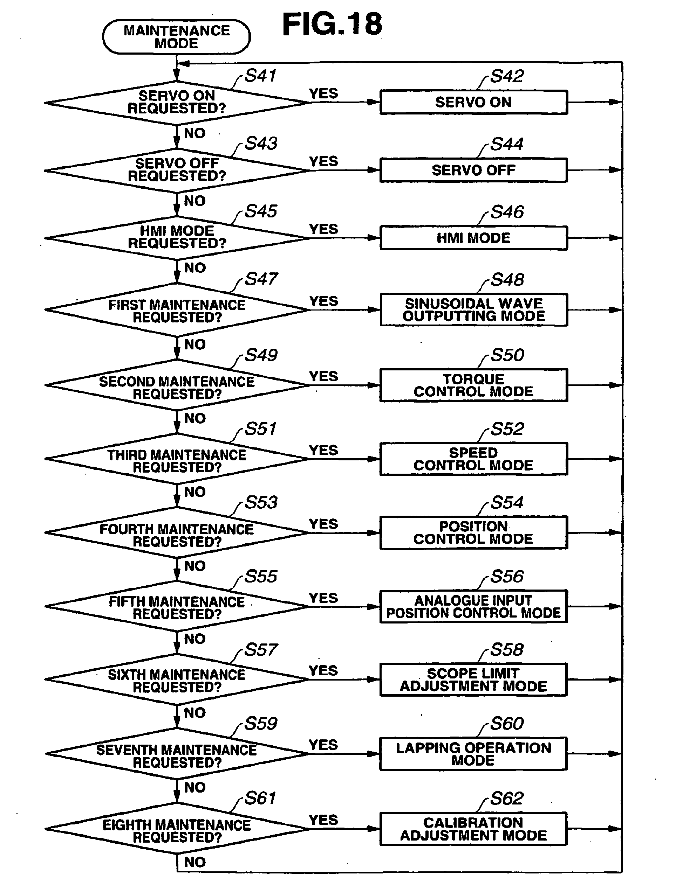

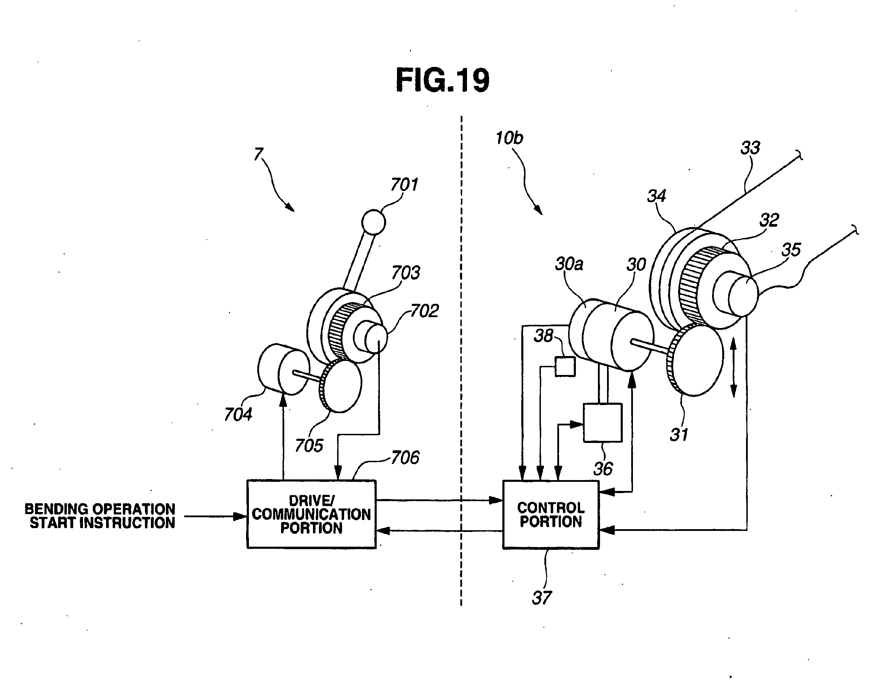

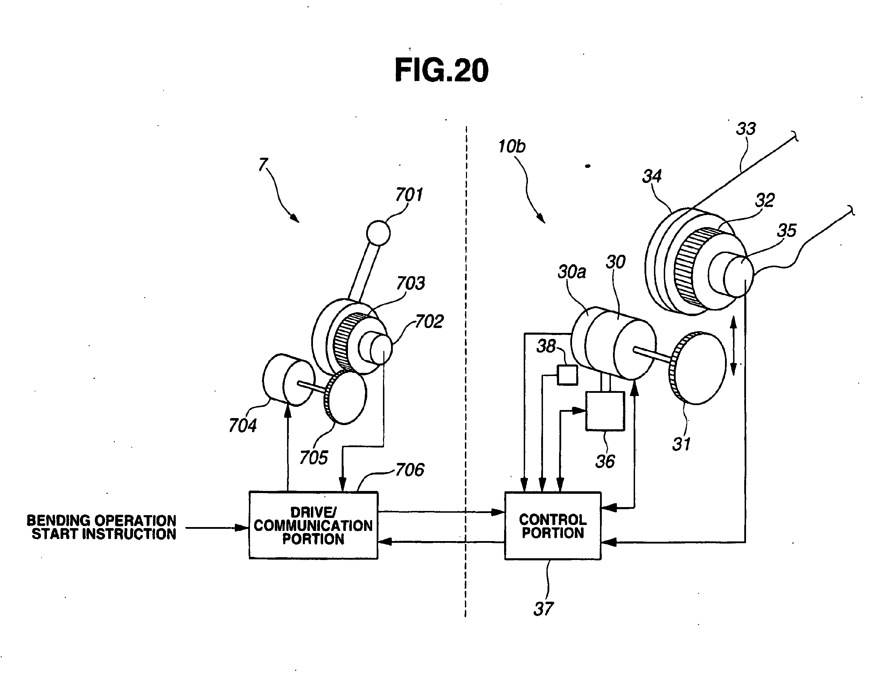

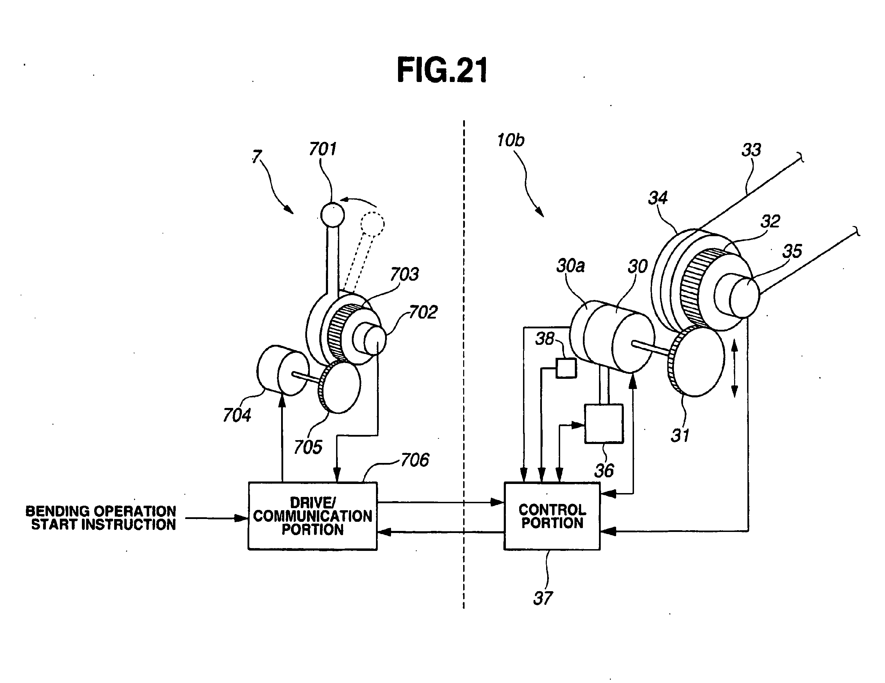

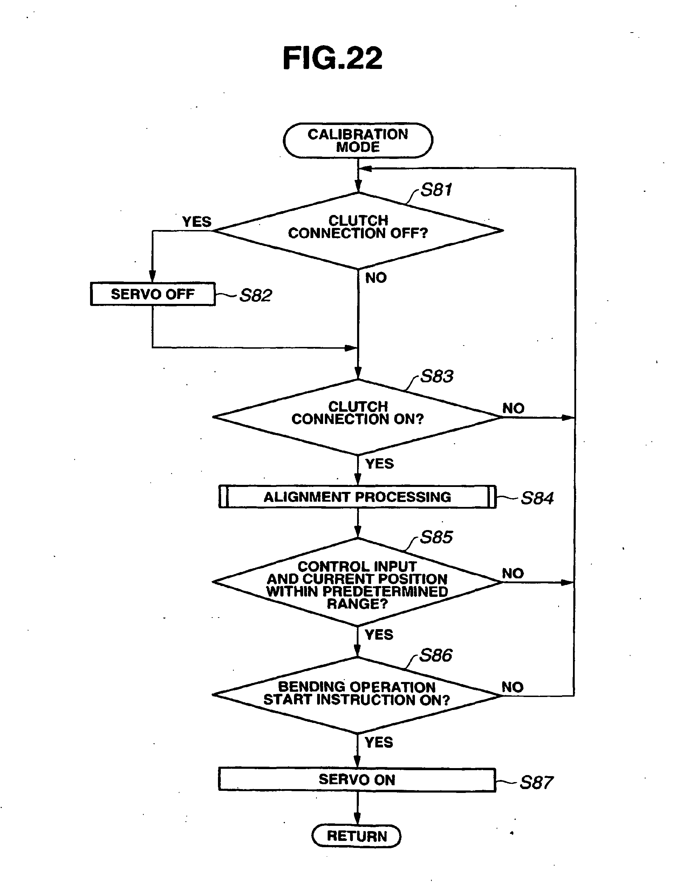

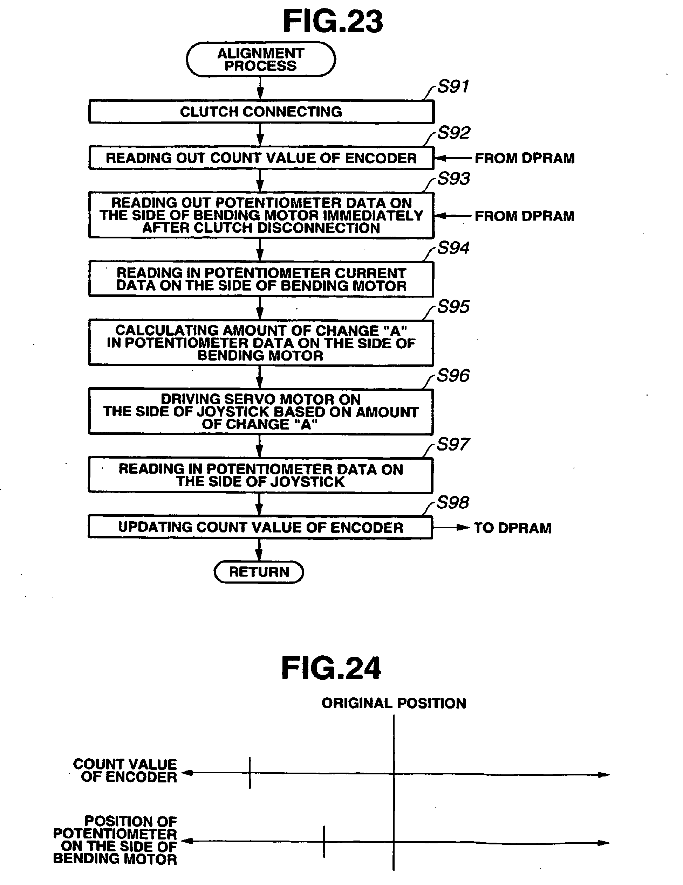

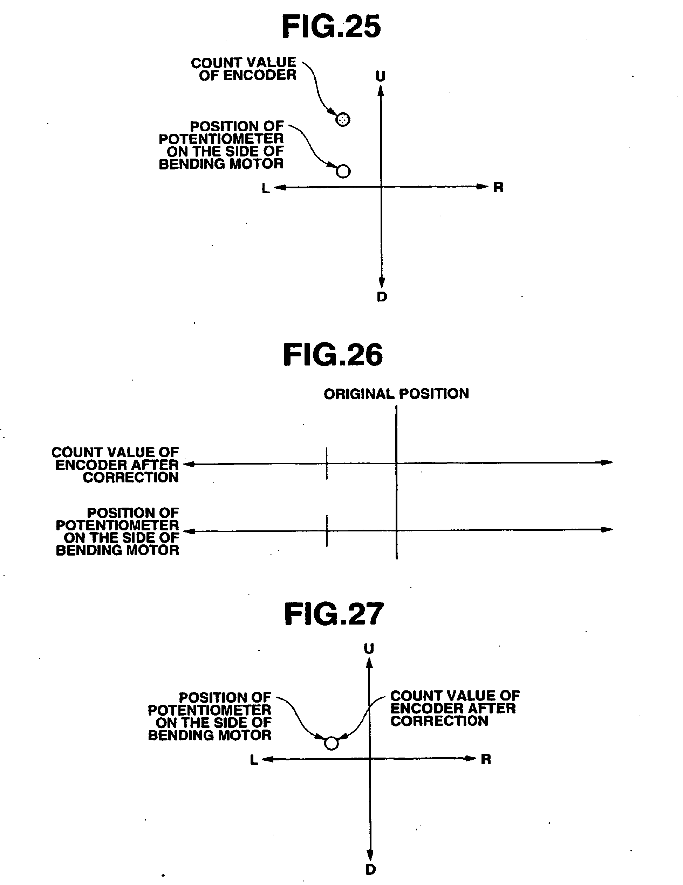

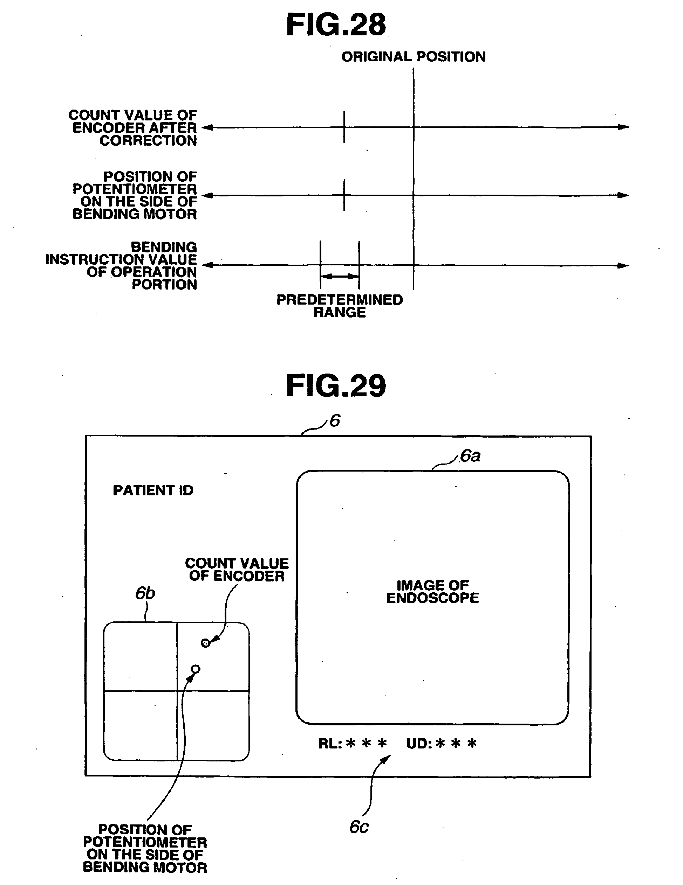

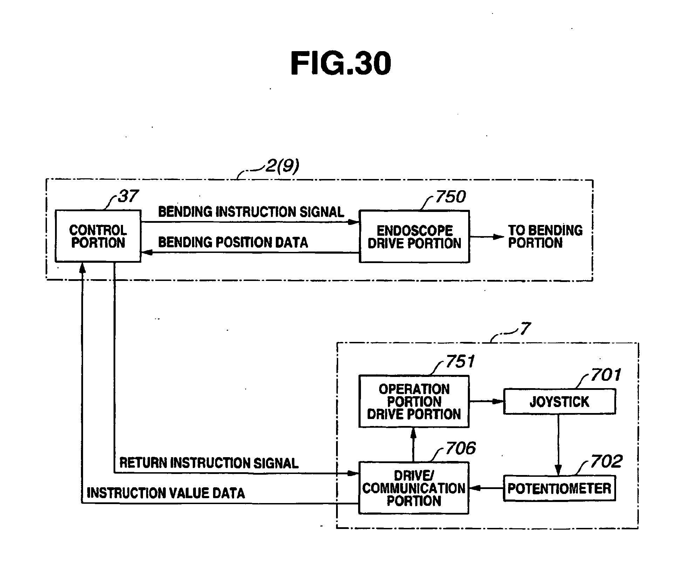

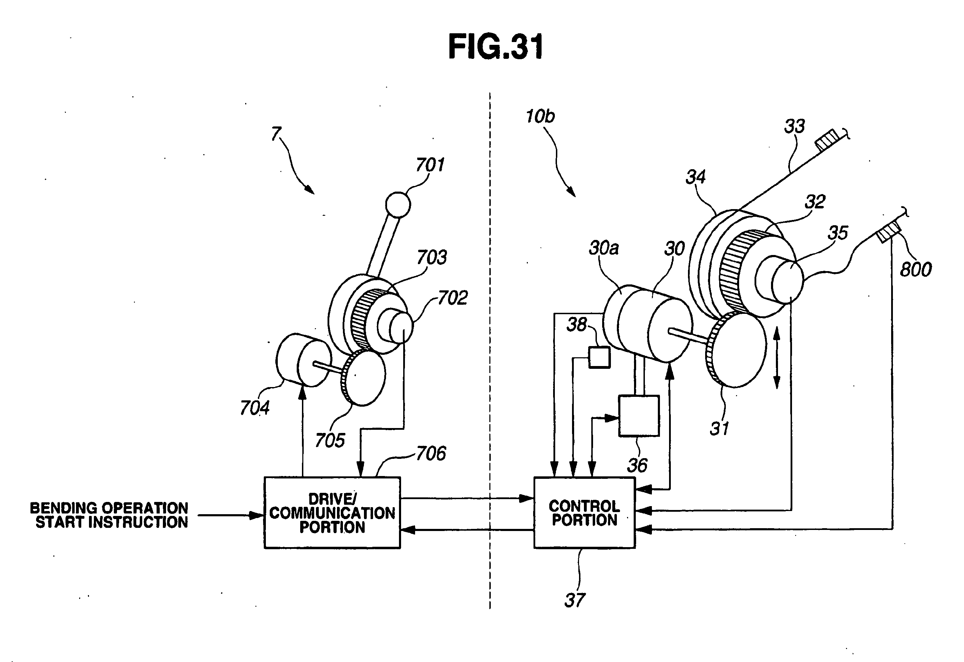

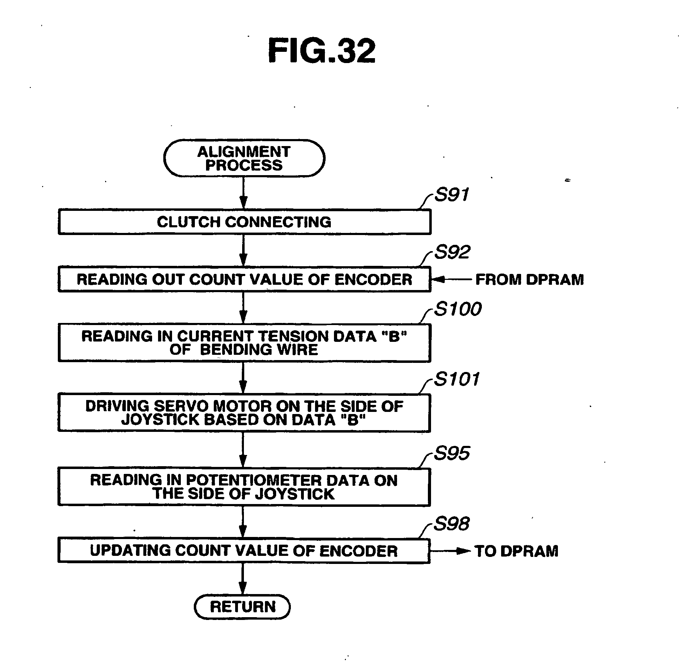

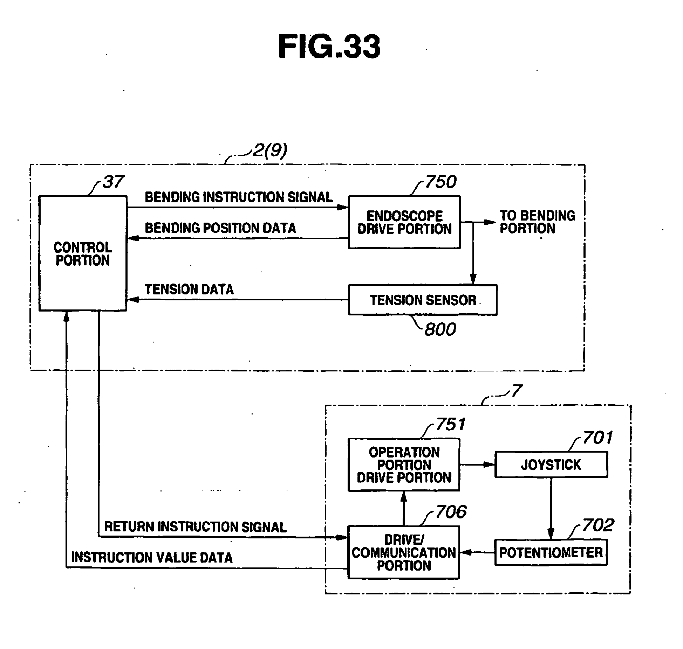

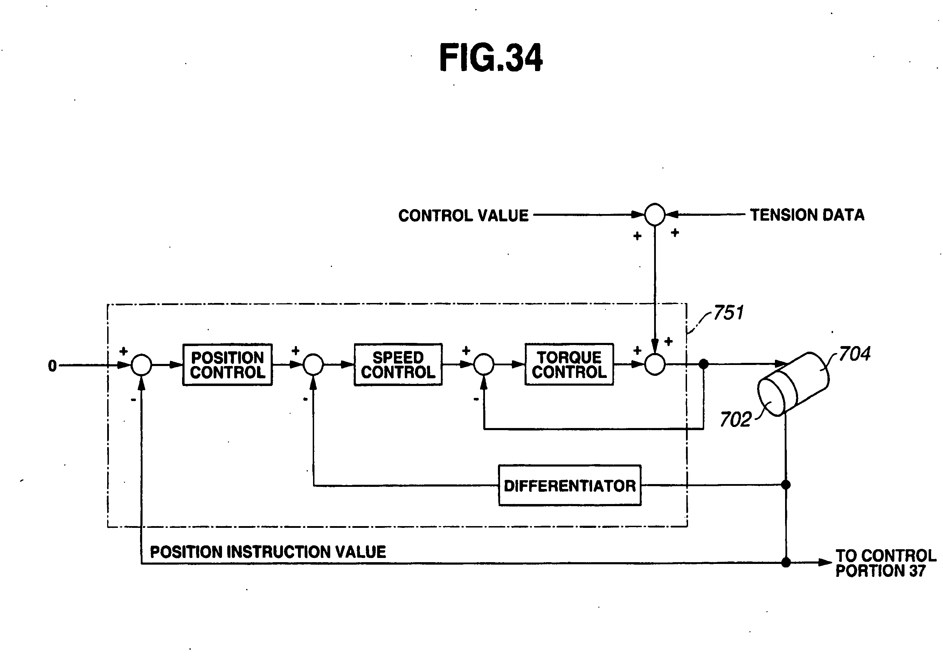

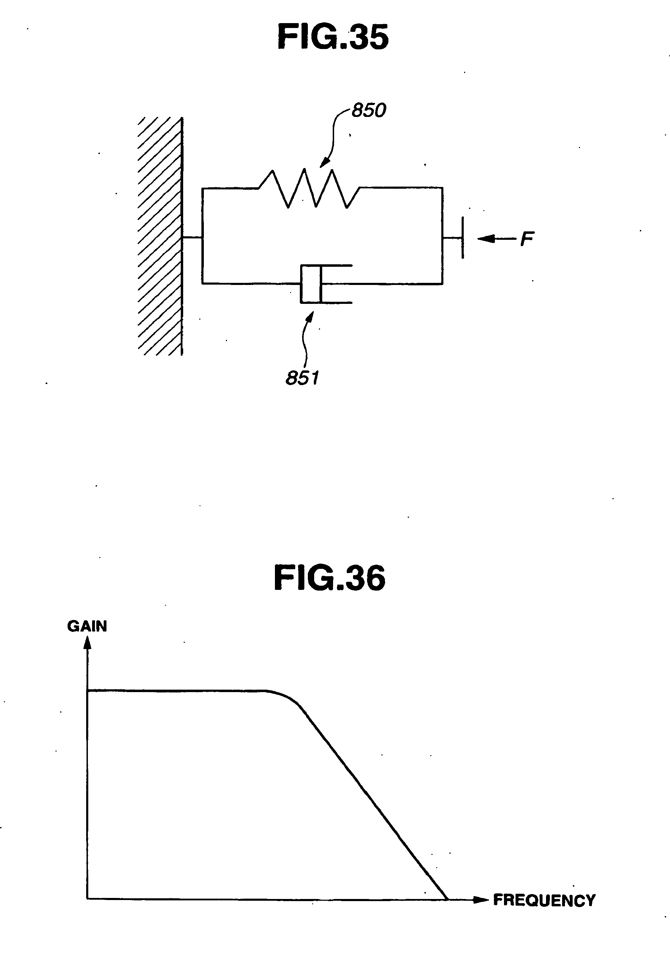

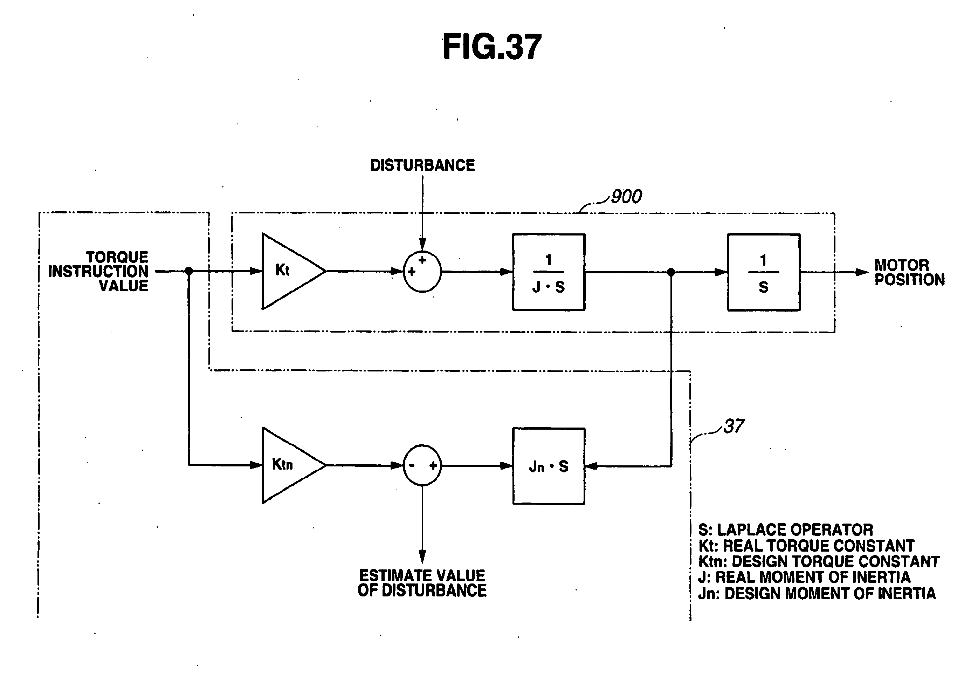

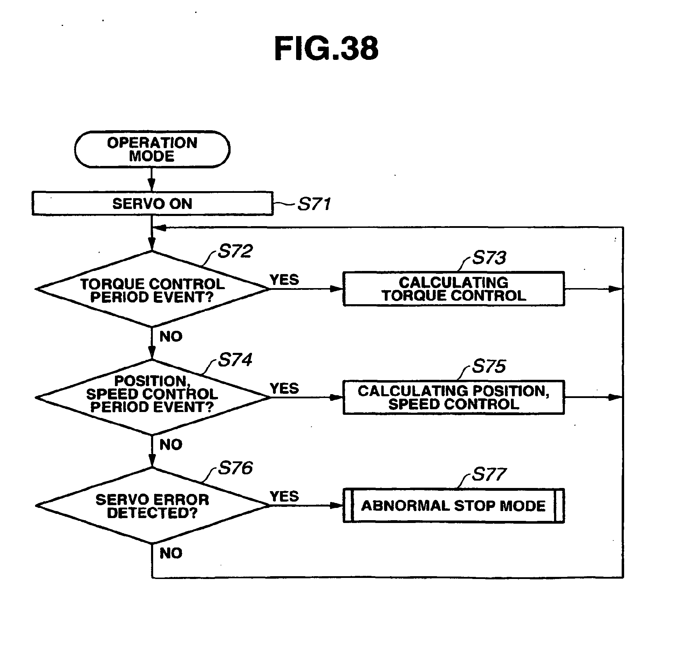

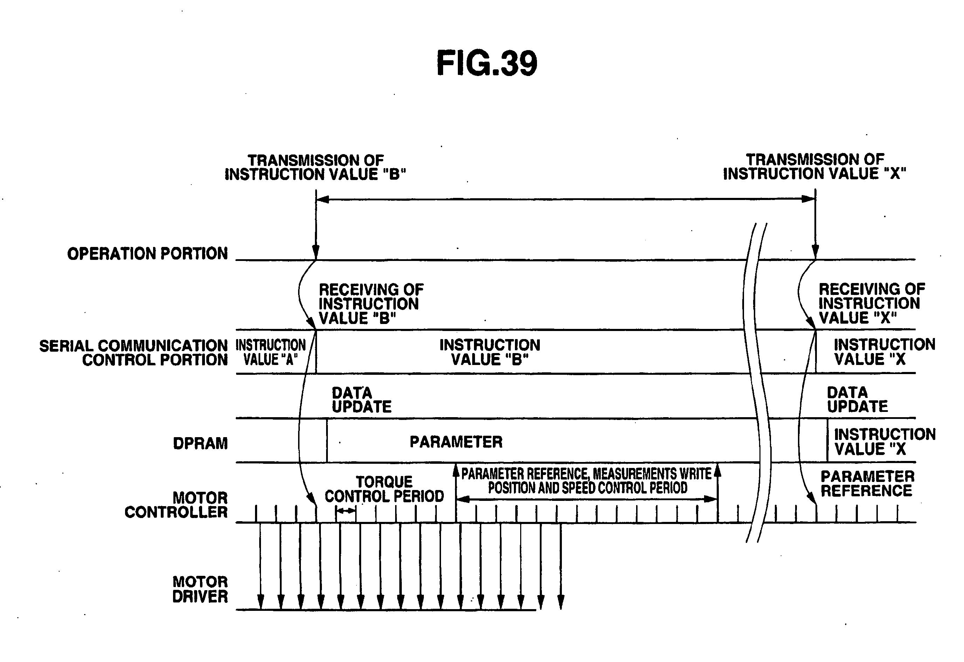

Figs. 1 to 39 are related to a first embodiment of the present invention. Fig. 1 shows a configuration diagram of an electric bending endoscope apparatus. Fig. 2 shows a configuration diagram of a front panel of an image processing device in Fig. 1. Fig. 3 shows a configuration diagram of a bending control portion in Fig. 1. Fig. 4 shows a configuration diagram of a control portion of the bending control portion in Fig. 1. Fig. 5 shows a configuration diagram of a logic block of an FPGA in Fig. 4. Fig. 6 shows a configuration diagram of a control processing portion of a motor controller in Fig. 5. Fig. 7 shows a configuration diagram of a servo error detection portion of the motor controller in Fig. 5. Fig. 8 is a diagram illustrating servo control of the motor controller in Fig. 5. Fig. 9 is a diagram illustrating a first variation of the servo control of the motor controller in Fig. 5. Fig. 10 is a diagram illustrating a variation of a configuration of the FPGA in Fig. 4. Fig. 11 is a diagram illustrating a second variation of the servo control of the motor controller in Fig. 5. Fig. 12 is a diagram illustrating a logic element block constituting an FPGA block error monitor portion in Fig. 5. Fig. 13 is a first diagram illustrating a logic determination block using the logic element block in Fig. 12. Fig. 14 is a second diagram illustrating the logic determination block using the logic element block in Fig. 12. Fig. 15 is a diagram illustrating process transition in the FPGA in Fig. 5. Fig. 16 is a flow chart illustrating processes in the FPGA in Fig. 5. Fig. 17 is a flow chart illustrating an initial mode process in Fig. 16. Fig. 18 is a flow chart illustrating a maintenance mode process in Fig. 16. Fig. 19 shows a configuration diagram of a remote control operation portion for the bending control portion on clutch connection in Fig. 3. Fig. 20 shows a configuration diagram of the remote control operation portion for the bending control portion on clutch disconnection in Fig. 3. Fig. 21 shows a configuration diagram of the remote control operation portion for the bending control portion on clutch reconnection in Fig. 3. Fig. 22 is a flow chart illustrating a calibration mode process in Fig. 16. Fig. 23 is a flow chart illustrating an alignment process in Fig. 22. Fig. 24 is a first view illustrating the alignment process in Fig. 22. Fig. 25 is a second view illustrating the alignment process in Fig. 22. Fig. 26 is a third view illustrating the alignment process in Fig. 22. Fig. 27 is a fourth view illustrating the alignment process in Fig. 22. Fig. 28 is a fifth view illustrating the alignment process in Fig. 22. Fig. 29 is a sixth view illustrating the alignment process in Fig. 22. Fig. 30 is a diagram illustrating the calibration mode process in Fig. 16 in terms of signal control. Fig. 31 shows a configuration diagram of a variation of the remote control operation portion for the bending control portion on clutch connection in Fig. 3. Fig. 32 is a flow chart illustrating the alignment process in a configuration in Fig. 30. Fig. 33 is a first view illustrating the calibration mode process in a configuration in Fig. 31 in terms of signal control. Fig. 34 is a second view illustrating the calibration mode process in the configuration in Fig. 31 in terms of signal control. Fig. 35 is a third view illustrating the calibration mode process in the configuration in Fig. 31 in terms of signal control. Fig. 36 is a fourth view illustrating the calibration mode process in the configuration in Fig. 31 in terms of signal control. Fig. 37 is a diagram illustrating creation of tension data substituting for means for sensing tension of an endoscope bending wire shown in Fig. 31. Fig. 38 is a flow chart illustrating an operation mode process in Fig. 16. Fig. 39 is a timing chart illustrating the operation mode process in Fig. 16.

An electric bending endoscope apparatus 1 of the present embodiment, as shown in Fig. 1, mainly includes: an electric bending endoscope (hereinafter, abbreviated to "endoscope") 2 in which, for example, an image pickup device (not shown) is embedded in a distal end rigid portion of an endoscope insertion portion (hereinafter, abbreviated to "insertion portion") 9, and a bending portion 11 of the insertion portion 9 is bent by electrically pulling a bending wire (to be described below) constituting bending drive means; a remote control operation portion 7 for performing drive operation and the like onto the bending portion 11; an image processing device 4 for creating a video signal from an image signal transmitted through a universal cable 12; a light source device 3 for providing illumination light to an illuminating optical system not shown through a light guide fiber bundle (not shown) included in the universal cable 12; a monitor 6 that is a display device to which the video signal created by the image processing device 4 is outputted, and which displays an image of the endoscope; and a pump unit 14 for air supply, water supply and suction.

The light source device 3, the image processing device 4 and the pump unit 14 are mounted on a cart 15, and in the pump unit 14, a flow control cassette 14a including a flow adjustment mechanism for air supply, a water supply and suction is detachably provided. Further, on the cart 15, an endoscope fixing arm 13 for holding and fixing the endoscope 2 is provided, and a proximal end grasping portion 10 of the endoscope 2 is removably held and fixed on a distal end of the endoscope fixing arm 13.

A forceps plug 10a to which a suction tube from the flow control cassette 14a can be connected is disposed on the proximal end grasping portion 10 of the endoscope 2, and the universal cable 12 and a tube for air supply and a tube for water supply from the flow control cassette 14a are connected to the proximal end grasping portion 10. The tubes for air supply and for water supply, a suction tube, and the like are linked with, for example, a conduit line for air supply, a conduit line for water supply, and a suction conduit line not shown included in the insertion portion 9.

Further, in the proximal end grasping portion 10, a bending control portion 10b for controlling a motor and the like to electrically drive the bending portion 11 is embedded, and the remote control operation portion 7 is connected to the bending control portion 10b through a cable 7a. In addition, the remote control operation portion 7 is capable of also being connected to the image processing device 4 through the cable 7a, and to the bending control portion 10b through the universal cable 12.

The remote control operation portion 7 includes an operation input device for performing electric bending operation of the bending portion 11 described below, for example, a joystick 701 as instruction means, and though not shown, and scope switches composed of operation input switches of air supply, water supply and suction, remote switches for freeze and release in the image processing device 4.

The image processing device 4 is adapted to be connected to the pump unit 14, and a front panel 4a of the image processing device 4, as shown in Fig. 2, includes: a power switch 20, an initialization button 23 having an LED function for instructing initialization of the electric bending endoscope apparatus 1 and indicating completion of the initialization; a calibration LED portion 24 for indicating calibration of electrical bending of the bending portion 11; a group of operation input switches 25 for air and water supply and suction of the pump unit 14; an examination-ready LED 26 for indicating a state that an examination with the electric bending endoscope apparatus 1 is ready; and a conduit line connection display portion 27 for displaying a connection state of the air supply conduit line, the water supply conduit line and the suction conduit line.

As shown in Fig. 3, into the insertion portion 9 are inserted an up and down bending wire 33, and a right and left bending wire not shown which extend from the bending control portion 10b to operate the bending portion 11 to bend. Note that, in the following description, a configuration regarding the up and down bending wire 33 will be described, but a configuration regarding the right and left bending wire configured similarly to the up and down bending wire 33 will not be shown and a description thereof will be also omitted for simplicity.

Both end portions of the bending wire 33 are linked and fixed to, for example, a chain not shown, and the chain is disposed to mesh with a rotatable sprocket portion 34 for up and down constituting bending drive means. Accordingly, rotation of the sprocket portion 34 in a predetermined direction pulls the bending wire 33 fixed to the chain, so that the bending portion 11 is operated to bend in a predetermined direction.

The sprocket portion 34, for example, is disposed in the bending control portion 10b. The sprocket portion 34 includes: a plurality of gears 31 and 32, to which is transmitted a drive force of an up and down bending motor 30 composed of, for example, a three-phase motor, as bending power means; and a clutch mechanism portion 36 which is drive force transmission disconnected and recovered means, for example, drive force transmission means for making the gears mesh or disengage with each other. Then, by the clutch mechanism portion 36, the bending wire 33 is released from a tension acting thereon, and thereby providing a state that the bending portion 11 can freely bend by an external force.

Herein, the bending drive means includes the gears 31 and 32, the bending wire 33, and the sprocket portion 34.

The clutch mechanism portion 36 is adapted to switch between the drive force transmission disconnected state that the clutch mechanism portion 36 is in disconnection and the drive force transmission recovered state that the clutch mechanism portion 36 is in connection by operating a selection operation lever 10c (see Fig. 1) which is state selecting means to switch between the drive force transmission disconnected position (hereinafter, referred to as "bending free instruction position"), and the drive force transmission recovered position (hereinafter, referred to as "angle operation instruction position").

That is, by operating the selection operation lever 10c to mechanically select a cutoff state or a connected state of the clutch mechanism portion 36, the bending motor 30 can be connected to and disconnected from the sprocket portion 34, interchangeably.

Rotation of the sprocket portion 34 is detected by a potentiometer 35 which is bending state detection means. A symbol 30a indicates an encoder which is drive state detection means for detecting rotation of the bending motor 30. Further, a symbol 38 indicates a thermistor for measuring a temperature of the bending motor 30.

A control portion 37 of the bending control portion 10b has the remote control operation portion 7, the encoder 30a, the potentiometer 35, the clutch mechanism portion 36 and the thermistor 38 connected thereto.

The bending control portion 10b, as shown in Fig. 4, includes: a power supply connector 50 to which a power supply cable (not shown) is connected through the universal cable 12; and an operation portion connector 51 to which the cable 7a of the remote control operation portion 7 is connected. The power supply connector 50 is connected to a controlling power supply portion 52 and a driving power supply portion 53 in the control portion 37. The controlling power supply portion 52 supplies power for control to each portion through a DC/DC converter 54. Further, the driving power supply portion 53 supplies drive power for a three-phase sinusoidal wave power generated by a motor driver 55.

The operation portion connector 51 is connected to a field programmable gate array (FPGA) 56 in the bending control portion 10b. This FPGA 56 is adapted to configure an internal cell into a desired logic block based on data stored in an EEPROM 59. The FPGA 56 has the encoder 30a, the potentiometer 35, the clutch mechanism portion 36 and the thermistor 38 connected thereto, and controls them. Further, the FPGA 56 supplies data to the motor driver 55 for generating the three-phase sinusoidal wave power, and the motor driver 55 accordingly supplies the three-phase sinusoidal wave power to the bending motor 30.

The FPGA 56 outputs a WDT-CR signal for clearing a watch dog timer (WDT) 57 when an error beyond a predetermined fixed level occurs in the internal cell. According to the WDT-CR signal, the WDT 57 outputs a reset signal to the FPGA 56, and the FPGA 56 is reset. The FPGA 56, when the reset signal is inputted, activates a reset IC 58 to cause reconfiguration based on the EEPROM 59, and reconfigures the logic block in the internal cell.

The logic block of the FPGA 56, as shown in Fig. 5, includes a serial communication unit 100, a serial communication control portion 101, an EEPROM controller 102, an error signal processing portion 103, an LED controller 104, an operation mode controller 105, a DPRAM 106, a clutch signal input portion 107, a jig board input output portion 108, a RAM 109, a motor controller 110, a motor drive waveform creation portion 111, an RL (right and left) motor current F/B portion 112, a UD (up and down) motor current F/B portion 113, a potentiometer control portion 114, a thermistor control portion 115, an RL encoder control portion 116, a UD encoder control portion 117, and an FPGA block error monitor portion 118. Further, the motor controller 110 includes each logic block of a measurement processing portion 200, a control processing portion 201, a servo error detection portion 202, and a servo ON/OFF control portion 203.

In Fig. 5, solid lines show a normal flow of control and data signals, and dotted lines indicate a flow of a logic block error signal, a servo error signal, and a communication error signal.

The serial communication unit 100 communicates with the remote control operation portion 7 in serial, for example, with LVDS, and the serial communication control portion 101 controls the serial communication unit 100, and communicates with the motor controller 110, and stores data received from the motor controller 110 in the DPRAM 106.

The EEPROM controller 102 executes configuration of the FPGA 56 according to a program stored in the EEPROM 59.

The error signal processing portion 103 monitors an abnormal power supply voltage and an overcurrent of the bending motor 30, and outputs the monitor result to the operation mode controller 105.

The clutch signal input portion 107 receives a state signal indicating the power transmission disconnected state or the drive force transmission recovered state from the clutch mechanism portion 36, and outputs the signal to the operation mode controller 105.

The jig board input output portion 108 exchanges data with a jig board (not shown) for debugging. Further, the LED controller 104 controls an LED of the jig board.

The operation mode controller 105 outputs an operation mode to the motor controller 110 corresponding to the power transmission disconnected state or the drive force transmission recovered state from the clutch mechanism portion 36, and a connection state with the jig board. The operation mode controller 105 is adapted to receive the communication error signal outputted from the serial communication control portion 101, and the servo error signal outputted from the motor controller 110, and to output the operation mode based on the error signals to the motor controller 110.

The motor drive waveform creation portion 111 reads out sinusoidal wave data stored in the RAM 109 through the motor controller 110, creates three-phase sinusoidal wave data, and outputs the three-phase sinusoidal wave data to the RL (right and left) motor driver and the UD (up and down) motor driver 55.

The RL (right and left) motor current F/B portion 112 converts a U-phase current value and a V-phase current value of the RL (right and left) motor into a digital signal, and outputs the signal to the motor controller 110. Similarly, the UD (up and down) motor current F/B portion 113 converts a U-phase current value and a V-phase current value of the UD (up and down) motor 30 into a digital signal, and outputs the signal to the motor controller 110.

The potentiometer control portion 114 converts position data of the potentiometer 35 connected to the RL (right and left) sprocket portion and the UD (up and down) sprocket portion 34 into a digital signal, and outputs the signal to the motor controller 110.

The thermistor control portion 115 converts temperature data measured by the thermistor 38 provided in the RL (right and left) motor and the UD (up and down) motor 30 into a digital signal, and outputs the signal to the motor controller 110.

The RL encoder control portion 116 and the UD encoder control portion 117 output a count value of the encoder 30a provided in the RL (right and left) motor and the UD (up and down) motor 30 to the motor controller 110.

Then, the motor controller 110 servo-controls the RL (right and left) motor and the UD (up and down) motor 30 based on the operation mode by using the measurement processing portion 200, the control processing portion 201, the servo error detection portion 202, and the servo ON/OFF control portion 203.

Further, the FPGA block error monitor portion 118 is adapted to receive the logic block error signal, the servo error signal, and the communication error signal of the each logic block, and to output a TRG signal to the motor controller 110 based on the error signals, and to output the WDT-CR signal to the WDT 57.

Here, the control processing portion 201 of the motor controller 110, as shown in Fig. 6, includes a position control block 201a, a speed control block 201b, and a torque control block 201c, and further, the servo error detection portion 202, shown in Fig. 7, includes a position deviation error determining block 202a, a rotation direction error detection block 202b, an abnormal speed detection block 202c, and an overload error detection block 202d.

Next, servo control of the motor controller 110 will be described referring to Fig. 8. The position control block 201a compares a position instruction value outputted from the remote control operation portion 7 with an output value of the encoder 30a, and the position deviation error determining block 202a outputs a servo error signal when the position deviation exceeds a predetermined value.

Further, the speed control block 201b compares an output of the position control block 201a with a differential value (obtained by a differentiating circuit 211) of the output value of the encoder 30a. The rotation direction error detection block 202b outputs a servo error signal, upon detecting an error of the rotation direction based on the output of the position control block 201 a and the differential value of the output of the encoder 30a. Further, the abnormal speed detection block 202c outputs a servo error signal, upon detecting a speed error based on the differential value of the output of the encoder 30a.

Further, the torque control block 201c compares an output of the speed control block 201b with the current value of the motor driver 55, and controls the motor driver 55. The overload error detection block 202d monitors an overload state of the bending motor 30 based on the output of the speed control block 201 b, and outputs a servo error signal, upon determining that the bending motor 30 is in an overload state.

Note that, when an error occurs in the position control block 201 a or the speed control block 201b, the FPGA block error monitor portion 118 can output a TRG signal to the motor controller 110 based on the logic block error signal to control a switch portion 210a, a switch portion 210b, or a switch portion 210c, and thereby omitting control of the position control block 201a or the speed control block 201b.

The servo control of the motor controller 110 may be executed in a manner, for example, that, as shown in Fig. 9, pairs of the position control blocks 201a, of the speed control blocks 201b and of the torque control blocks 201c are provided in parallel, respectively, and the switch portions 210a to 210f are controlled by the TRG signal to select a normal control block for servo-control (a feedback system is omitted in Fig. 9 though).

Further, another way may be that, as shown in Fig. 10, two EEPROMs 59 are provided, and different programs in error processing and correcting methods are stored in these EEPROMs 59, and a selection determining portion 220 selects a program most suitable for processing and correcting an error by switching a switch portion 221 based on the error, and the selected program executes reconfiguration of the FPGA 56.

Further, still another way may be that, as shown in Fig. 11, the output of the encoder 30a and the output of the potentiometer 35 can be switched in the switch portion 222, and when an error occurs in the encoder 30a, the position control is performed based on the output of the potentiometer 35, and when an error occurs in the potentiometer 35, the position control is performed based on the output of the encoder 30a.

The FPGA block error monitor portion 118 creates the WDT-CR signal or the TRG signal, by using, as one example, a logic determination block 251, as shown in Fig. 13, having a plurality of logic element blocks 250 composed of an AND, an OR, and a switch as shown in Fig. 12.

That is, as shown in Fig. 14, a plurality of the logic determination blocks 251(1) to 251(n) determines an error based on a plurality of error factors as conditions, and the appropriate determination creates an appropriate TRG signal or a WDT-CR signal timed appropriately.

Operation of the present embodiment configured in such a manner will be described. In the present embodiment, as shown in Fig. 15, when power is turned on, first, an initial mode process is executed. After the initial mode process, then the process moves to a mode selecting process.

Here, an operation mode is a mode in which operation of electrical bending is performed based on an operation instruction from the remote control operation portion 7, and a maintenance mode is a mode in which parameters are set (read and write), and in which remote operation for state monitoring and the like is performed with a dedicated jig or in an HMI mode connected to a personal computer as described below.

In the mode selecting process, for example, the process proceeds a calibration mode when the clutch is disconnected, or when a bending operation start instruction is off on completion of the initial mode process, and the process returns the mode selecting process when the clutch is connected again, and an operation instruction value and a scope position coincide with each other, or when the bending operation start instruction is on.

Further, in the mode selecting process, when the operation mode is selected, the process proceeds to the operation mode and the servo is turned on, and when the operation mode is instructed to end, the process returns to the mode selecting process.

Further, in the mode selecting process, when the maintenance mode is selected, the process proceeds to the maintenance mode and the servo is turned on, and when the maintenance mode is instructed to end, the process returns to the mode selecting process.

Also, in the mode selecting process, the process proceeds to an abnormal stop mode and the servo is turned off when a factor for requiring stop occurs.

The above contents will be described in detail referring to the flow chart in Fig. 16. When power is turned on, at step S1, the EEPROM controller 102 configures the FPGA 56. Subsequently, at step S2, the initial mode process (to be described below) is executed, and at step S3, the process waits for completion of the initial mode process.

After completion of the initial mode process, at step S4, the operation mode controller 105 issues a calibration request. Then, at step S5, it is determined whether or not the operation mode controller 105 issues a maintenance mode process request. When the maintenance mode process request is issued, at step S6, the maintenance mode process (to be described below) is executed, and the process returns to step S5.

When the maintenance mode process request is not issued, at step S7, it is determined whether or not the operation mode controller 105 returns from the maintenance mode process to the mode selecting process. Then, when the operation mode controller 105 returns to the mode selecting process, at step S8, the operation mode controller 105 issues the calibration request, and the process returns to step S5.

When the operation mode controller 105 does not return to the mode selecting process, at step S9, the operation mode controller 105 determines whether or not the calibration request is valid, and when the calibration request is valid, at step S10, the operation mode controller 105 executes the calibration process, and at step S 11, it is determined whether or not the calibration process is successfully completed. When the calibration process is not successfully completed, the process returns to step S5, and when the calibration process is successfully completed, at step S12, the calibration request is released, and the process returns to step S5.

When it is determined that the calibration request is not valid at step S9, at step S 13, the operation mode controller 105 determines whether or not the bending operation start instruction is turned off. When it is determined that the bending operation start instruction is turned off, at step S14, the operation mode controller 105 issues the calibration request, and the process returns to step S5.

When it is determined that the bending operation start instruction is not turned off, at step S 15, the operation mode controller 105 determines whether or not the clutch connection is off. When the clutch connection is off, the process proceeds to step S14, and when the clutch connection is on, at step S16, the operation mode process is executed (to be described below), and the process returns to step S5.

Next, referring to the flow chart in Fig. 17, the initial mode process will be described. At step S21, first, the WDT 57 starts. Then, at step S22, each logic block initializes an internal parameter, and at step S23, the RL (right and left) motor current F/B portion 112, the UD (up and down) motor current F/B portion 113, the potentiometer control portion 114, and the thermistor control portion 115 start to sample data, respectively.

Then, at step S24, the serial communication unit 100 and the serial communication control portion 101 start communicating, and at step S25, it is determined whether or not external hardware is normal, and when abnormal, at step S26, the abnormal stop mode process is executed.

When it is determined that the external hardware is normal, at step S27, the motor controller 110 determines whether or not an offset of the motor current is normal, and when the offset of the motor current is abnormal, at step S26, the abnormal stop mode process is executed.

Then, when it is determined that the offset of the motor current is normal, at step S28, the motor controller 110 detects a rotor position of the motor 30, and at step S29, reads in parameters in the DPRAM 106.

Next, at step S30, the motor controller 110 determines whether or not values of the parameters read in are all "0", and when the values of the parameters are not all "0", the process is completed as it is, and when the values of the parameters are all "0", at step S31, the motor controller 110 writes defaults of the parameters in the DPRAM 106, and ends the process.

Next, referring to the flow chart in Fig. 18, the maintenance mode process will be described. The operation mode controller 105 starts to communicate with the jig (not shown), and at step S41, the operation mode controller 105 determines whether or not the jig issues a servo ON request, and at step S42, when the servo ON request is issued, the servo is turned on, and the process returns to step S41.

Similarly at step S41, the operation mode controller 105 determines whether or not the jig issues a servo off request, and at step S44, when the servo off request is issued, the servo is turned off, and the process returns to step S41.

Next, at step S45, the operation mode controller 105 determines whether or not the jig issues an HMI mode (the servo state monitor mode) request, and at step S46, when the HMI mode request is issued, an HMI mode process is executed, and the process returns to step S41.

Next, at step S47, the operation mode controller 105 determines whether or not the jig issues a first maintenance request, and at step S48, when the first maintenance request is issued, a sinusoidal wave output mode process is executed, and the process returns to step S41.

Subsequently, at step S49, the operation mode controller 105 determines whether or not the jig issues a second maintenance request, and at step S50, when the second maintenance request is issued, a torque control mode process is executed, and the process returns to step S41.

Next, at step S51, the operation mode controller 105 determines whether or not the jig issues a third maintenance request, and at step S52, when the third maintenance request is issued, a speed control mode process is executed, and the process returns to step S41.

Then, at step S53, the operation mode controller 105 determines whether or not the jig issues a fourth maintenance request, and at step S54, when the fourth maintenance request is issued, a position control mode process is executed, and the process returns to step S41.

Next, at step S55, the operation mode controller 105 determines whether or not the jig issues a fifth maintenance request, and at step S56, when the fifth maintenance request is issued, an analogue input position control mode process is executed, and the process returns to step S41.

Further, at step S57, the operation mode controller 105 determines whether or not the jig issues a sixth maintenance request, and at step S58, when the sixth maintenance request is issued, a scope limit adjustment mode process is executed, and the process returns to step S41.

Subsequently, at step S59, the operation mode controller 105 determines whether or not the jig issues a seventh maintenance request, and at step S60, when the seventh maintenance request is issued, a lapping operation mode process is executed, and the process returns to step S41.

Here, a lapping operation mode is one in which a predetermined bending operation, for example, a sequential operation such as RL->UD->RL is performed.

Next, at step S61, the operation mode controller 105 determines whether or not the jig issues an eighth maintenance request, and at step S62, when the eighth maintenance request is issued, a calibration adjustment mode process is executed, and the process returns to step S41.

As described above, with respect to each function necessary for the electric bending operation, its functional operation can be separately confirmed.

Next, referring to Figs. 19 to 37, the calibration mode process executed by the operation mode controller 105 will be described. As shown in Fig. 19, the remote control operation portion 7 includes a joystick 701, whose position is detected by a potentiometer 702. Further, the joystick 701 includes a gear 703, and by engagement of the gear 703 with a gear 705 provided in a rotation axis of a servo motor 704 as instruction drive means, a drive force of the servo motor 704 can move the joystick 701. Further, the remote control operation portion 7 includes a drive/communication portion 706 which detects position data of the potentiometer 702, drives the servo motor 704, and is capable of communicating with the control portion 37.

Here, instruction drive control means includes, for example, the potentiometer 702 and the drive/communication portion 706.

As shown in Fig. 20, in the bending control portion 10b, clutch operation in the clutch mechanism portion 36 allows the gear 32 to be disconnected from the gear 31. When the gear 32 is disconnected from the gear 31, the bending wire 33 becomes free, and an output value of the potentiometer 35 becomes independent of a bending instruction value of the joystick 701. When the clutch is disconnected, the output value of the potentiometer 35 at the time of disconnection is stored in the DPRAM 106, and the output value of the potentiometer 35 in a free state and a count value of the encoder 30a are monitored, and their latest values are stored in the DPRAM 106.

In such situations, as shown in Fig. 21, when the clutch operation in the clutch mechanism portion 36 engages the gear 31 with the gear 32 again, the calibration mode process described above, that is, adjustment between the joystick 701 and the bending portion 11 becomes necessary.

Therefore, as shown in Fig. 22, at step S81, the operation mode controller 105 determines whether or not the clutch connection is off, and when the clutch connection is off, at step S82, the servo is turned off, and the process proceeds to step S83, and when the clutch connection is not off, the process proceeds directly to step S83.

Then, at step S83, the operation mode controller 105 determines whether or not the clutch connection is on, and when the clutch connection is on, the process proceeds to step S84, and when the clutch connection is not on, the process returns to step S81.

At step S84, an alignment process (to be described below) is executed, and subsequently, at step S85, it is determined whether or not an operation amount and a current position are within a predetermined range, and when within the predetermined range, the process proceeds to step S86, and when not within the predetermined range, the process returns to step S81.

Then, at step S86, it is determined whether or not the bending operation start instruction is on, and when the bending operation start instruction is on, at step S87, the servo is turned on, and the process is ended, and when the bending operation start instruction is not on, the process returns to step S81.

The alignment process described above, as shown in Fig. 23, is that, when the clutch connection is confirmed at step S91, a current count value of the encoder 30a is read out from the DPRAM 106 at step S92, and the output value of the potentiometer 35 obtained immediately after the clutch disconnection is read out from the DPRAM 106 at step S93. Fig. 24 schematically shows a difference immediately after the clutch connection between, for example, a current count value of the encoder 30a on the UD side, and the output value of the potentiometer 35, and further, Fig. 25 shows the count values of the UD and RL encoders, and the output value of the potentiometer in a two-dimensional diagram.

Then, at step S94, a current output value of the potentiometer 35 is read out, and at step S95, an amount of change A is calculated from the difference between the output value of the potentiometer 35 obtained immediately after disconnection and the current output value of the potentiometer 35.

Next, at step S96, the drive/communication portion 706 is controlled to drive the servo motor 704 on the side of the joystick 701 based on the amount of change A. At this time, the motor 30 of the bending portion is not driven and remains stopped.

Then, at step S97, the value of the potentiometer 702 on the side of the joystick 701 is read in, and at step S98, the current count value of the encoder 30a is updated in the DPRAM 106 to a value corresponding to the position of the potentiometer 702 on the side of the joystick 701, and the process is ended. Fig. 26 shows schematically shows the current count value of the encoder 30a, for example, on the UD side immediately after update, and the output value of the potentiometer 35, and further, Fig. 27 shows the count values of the UD and RL encoders, and the output value of the potentiometer in a two-dimensional diagram.

In such a manner, the adjustment between the count value of the encoder 30a and the output value of the potentiometer 35 is carried out, and the joystick 701 is moved by the servo motor 704, but as shown in Fig. 28, a bending instruction value issued by the joystick 701 has an error from the output value of the potentiometer 35 of the motor 30 of the bending portion, but it is assessed that, if the error range is within a predetermined range at step S85 in Fig. 22, the joystick 701, as shown in Fig. 28, can control the motor 30 of the bending portion.

In the present embodiment, as shown in Fig. 29, the monitor 6 displays, at the time of calibration, in addition to an image display area 6a for displaying an image of the endoscope, a navigation display area 6b for displaying a navigation image to adjust between the count value of the encoder 30a and the output value of the potentiometer 35, and a digital display area 6c for displaying the current output value of the tension sensor 35. Conventionally, only the digital display area 6c has been displayed and the joystick 701 has been manually adjusted.

As mentioned above, referring to Figs. 19 to 29, the calibration mode process has been described from the viewpoint of mechanical control, and referring to Fig. 30, the calibration mode process will be described then from the viewpoint of signal control.

In Fig. 30, an endoscope drive portion 750 includes the bending motor 30, the gears 31 and 32, the encoder 30a, the potentiometer 35 and the clutch mechanism portion 36. Further, an operation portion drive portion 751 includes the servo motor 704 and the gears 703 and 705.

Referring to Fig. 30, in the calibration mode, on the side of the motor controller 110, data of a current bending position is obtained from the endoscope drive portion 750. The data is translated into a position scale of the operation portion 7 in the control portion 37 of the controller 110.

The control portion 37, usually, translates an operation portion instruction (instruction value data) from the operation portion 7 into a position scale to the endoscope drive portion 750, thereby providing an endoscope drive portion instruction (bending instruction signal) having scale conversion applied thereto so that a movable range of the joystick 701 and a movable range of bending are coincided with each other. However, in the calibration mode, the endoscope drive portion instruction (bending instruction signal), in contrast, is translated to create translated data of the operation portion position scale.

Then, the translated data of the operation portion position scale are transferred to the operation portion 7 as return instruction data, and the operation portion drive portion 751 in the operation portion 7 moves to a return data position. The operation portion 7 accordingly can be automatically coincided with the bending portion.

Next, a variation of the calibration mode process will be described referring to Figs. 31 to 33. The calibration described above has been performed based on the output value of the potentiometer 35 of the motor 30 in the bending portion, but the case is not limited to this.

Fig. 31 shows an embodiment in which a tension sensor for detecting an amount of tension is disposed in the bending wire portion to bend the endoscope in the configuration of Fig. 21. Here, a configuration is not shown in which the tension sensor is disposed in the wire.

For example, as shown in Fig. 31, a tension sensor 800 may detect a tension state of the bending wire 33, and the joystick 701 may be moved by the drive force of the servo motor 704 based on the detected tension state of the bending wire 33 to align the joystick 701 with the bending position.

Specifically, as shown in Fig. 32, the clutch connection is confirmed at step S91, and the current count value of the encoder 30a is read out from the DPRAM 106 at step S92, and current tension data B of the bending wire 33 is read in through the tension sensor 800 at step S100.

Then, at step S101, the drive/communication portion 706 is controlled, and the servo motor 704 on the side of the joystick 701 is driven based on the tension data B. At this time, the motor 30 of the bending portion is not driven, and remains stopped.

Subsequently, at step S97, the output of the potentiometer 702 on the side of the joystick 701 is read in, and at step S98, the current count value of the encoder 30a is updated in the DPRAM 106 to a value corresponding to the position of the potentiometer 702 on the side of the joystick 701, and the process is ended.

As described above, the variation of the calibration mode process has been described from the viewpoint of mechanical control with reference to Figs. 31 and 32, and as shown in Fig. 30, the variation of the calibration mode process described above will be described from the viewpoint of signal control referring to Fig. 33 to Fig. 39.

In Fig. 33, a point different from Fig. 30 is that a tension sensor 800 is provided, and a tension state of the wire for pulling the endoscope is transferred to the controller.

Fig. 34 shows a block diagram with respect to the operation portion drive portion 751 in the operation portion 7. Since the illustration is provided by using the block diagram, and differs from an actual physical configuration, a supplementary explanation is added. A force input which an operator applies to tilt the joystick 701 is a control value in Fig. 34, and in response to an instruction of the operator by the joystick 701, a position instruction value is changed. At this time, because of a feedback loop configuration used for motor control, there are dynamic characteristics, and accordingly the joystick 701 has mechanical impedance. The dynamic characteristics are ones generally known such as a spring 850 and a damper 851 as shown in Fig. 35, and a frequency characteristic from the position instruction value and the operation value to the potentiometer 702 is a lowpass filter as shown in Fig. 36.

In addition to this, the tension data is configured to be superimposed on the operation value, and thereby a configuration can be provided in which a state of the endoscope insertion portion returns to the operator as feedback of force.

At this time, the tension data is set so that a reaction force responds to an instruction of the joystick 701. That is, in response to a direction in which the joystick 701 is tilted, a load to the wire for pulling the endoscope is configured to increase.

In addition, other than the tension sensor 800, the tension may be indirectly sensed by sensing an electrical current in the endoscope drive portion. This can be realized by sensing with a disturbance observer, as shown in Fig. 37 illustrating the principle of tension sensing in the insertion portion of the endoscope by sensing the electrical current.

In Fig. 37, a portion 900 shown by two-dot dash lines is a block diagram of a motor model of the actual bending motor 30. In Fig. 37, a torque instruction value from the motor driver is an electrical current instruction, and in response to this electrical current instruction, rotation and positioning of the bending motor 30 are set. Further, a disturbance shown as an input to the portion 900 of the two-dot dash lines is a disturbance load acting on a motor shaft. An approach to obtaining an estimate value of the disturbance is that the same physical model as the actual bending motor 30 shown by the portion 900 of the two-dot dash lines is, as shown in Fig. 37, disposed in parallel in the control portion 37, and the disturbance acting on the motor shaft is estimated from information about inverse dynamics to information about the torque instruction value and a number of revolution (speed) of the bending motor 30.

Next, referring to the flow chart in Fig. 38 and the timing chart in Fig. 39, the operation mode process will be described. First, at step S71, the servo is turned on, and at step S72, it is determined whether or not it is torque control period event time, and when it is the torque control period event time, a torque control calculation process is executed at step S73, and the process returns to step S72, and when it is not the torque control period event time, the process proceeds to step S74.

At step S74, it is determined whether or not it is position and speed control event time, and when it is the position and speed control event time, a position and speed control calculation process is executed at step S75, and the process returns to step S72, and when it is not the position and speed control event time, and the process proceeds to step S76. Then, at step S76, it is determined whether or not a servo error is detected, and when the servo error is detected, an abnormal stop mode process is executed at step S77, and when the servo error is not detected, the process returns to step S72.

As mentioned above, according to the present embodiment, in the remote control operation portion 7, the position of the joystick 701 is detected by the potentiometer 702, and the joystick 701 has the gear 703 provided therein, and the engagement of the gear 703 with the gear 705 provided in the rotation shaft of the servo motor 704 allows the joystick 701 to be moved by the drive force of the servo motor 704. Further, the position information of the potentiometer 702 is detected, the servo motor 704 is driven, and the drive/communication portion 706 capable of communicating with the control portion 37 is provided. Such a configuration allows the position of the joystick 701 to be automatically adjusted to the bending position of the bending portion even if switching between connection and disconnection of the clutch is carried out.

Note that, the control portion 37 has been provided in the bending control portion 10b of the endoscope 2, but the case is not limited to this, and the control portion 37 may be provided in the image processing device 4, or in a separate controller device.

### [Second Embodiment]

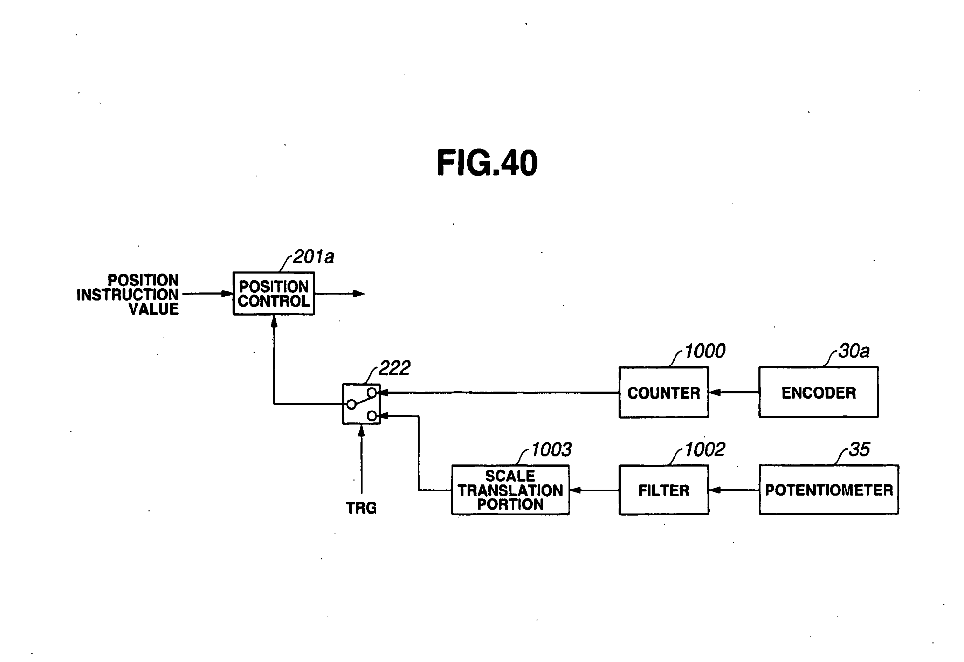

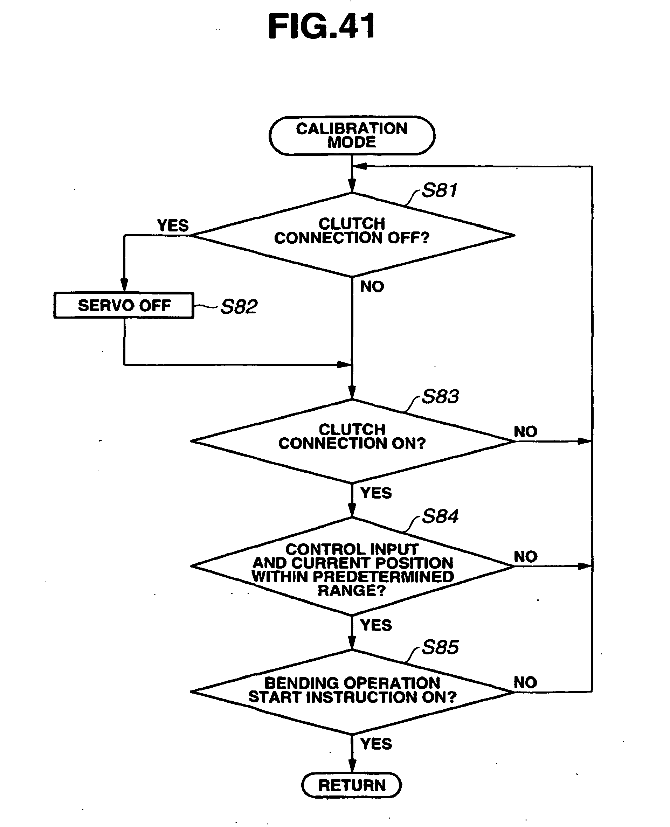

Figs. 40 and 41 relate to a second embodiment of the present invention, and Fig. 40 is a diagram illustrating position servo control of the motor controller of an FPGA, and Fig. 41 is a flow chart illustrating the calibration mode process in the position servo control in Fig. 40.

The second embodiment is almost similar to the first embodiment, and only a different point will be described, a like component is indicated by a like symbol, and a description thereof will be omitted.

In the present embodiment, as shown in Fig. 40, a switch portion 222 switches between the output of the encoder 30a and the output of the potentiometer 35, and when the clutch is off in the clutch mechanism portion 36, the output of the potentiometer 35 passing through a filter 1002 is converted into a position signal by a scale translation portion 1003, and the position signal is used for position control as an absolute position signal after passing through the switch portion 222. Also, when the clutch is on in the clutch mechanism portion 36, the output of the encoder 30a is counted by a counter 1000, and the counted position signal is used for position control as the absolute position signal after passing through the switch portion 222.

That is, in the calibration mode, rough position alignment is performed by using the value of the potentiometer 35, and in the operation mode, precise position control is performed by using the encoder 30a.

Consequently, control is realized in which the position of the joystick in the remote control operation portion 7 and the bending position strictly coincide with each other. The other configuration is the same as that of the first embodiment.

Operation of the present embodiment configured in such a manner will be described. Referring to the flowchart in Fig. 41, the calibration mode process of the present embodiment will be described.

At step S81, the operation mode controller 105 determines whether or not the clutch connection is off, and when the clutch connection is off, at step S82, the servo is turned off, and the process proceeds to step S83, and when the clutch connection is not off, directly the process proceeds to step S83.

Then, at step S83, the operation mode controller 105 determines whether or not the clutch connection is on, and when the clutch connection is on, the process proceeds to step S84, and when the clutch connection is not on, the process returns to step S81.

At step S84, it is determined whether or not the operation amount and the current position are within a predetermined range, and when within the predetermined range, the process proceeds to step S85, and when not within the predetermined range, the process returns to step S81.

Then, at step S85, it is determined whether or not the bending operation start instruction is on, and when the bending operation start instruction is on, the process is ended, and when the bending operation start instruction is not on, the process returns to step S81. The other operation is the same as that of the first embodiment.

As described above, in the present embodiment, when the clutch connection is off in the clutch mechanism portion 36, the output of the potentiometer 35 passing through the filter 1002 is converted into the position signal by the scale translation portion 1003, and the position signal is used for position control as the absolute position signal after passing through the switch portion 222. Further, when the clutch connection is on in the clutch mechanism portion 36, the output of the encoder 30a is counted by the counter 1000, and the counted position signal is used for position control as the absolute position signal after passing through the switch portion 222. Consequently, an appropriate bending drive control can be provided in response to a state in which the drive force is transmitted to the bending portion.

Note that, the control portion 37 has been provided in the bending control portion 10b of the endoscope 2, but the case is not limited to this, and the control portion 37 may be provided in the image processing device 4, or also in a separate controller device.

The present invention is not limited to the embodiments described above, and various changes and modifications may be made thereto without departing from the spirit and scope of the invention.

## Claims

1. An electric bending endoscope (2), comprising:
a bending portion (11) provided in an insertion portion (9);
bending drive means (31, 32, 33, 34) having a plurality of components configured to bend the bending portion (11);
bending power means (30) configured to output a drive force to drive the bending drive means (31, 32, 33, 34);
a motor controller (110) configured to control the bending power means (30) and read values from and write values into a DPRAM (106);
drive force transmission means (36) configured to selectively transmit the drive force from the bending power means (30) to the bending drive means (31, 32, 33, 34);
drive state detection means (30a) configured to detect information about a drive state of the bending power means (30);
bending state detection means (35) configured to sense operation information of the bending drive means (31, 32, 33, 34) to detect information about a bending state of the bending portion (11);
instruction means (701) configured to output bending instruction information to bend the bending portion (11);
a potentiometer (702) configured to detect position data of the instruction means (701);
instruction drive means (704) configured to drive the instruction means (701);
instruction drive control means (706) configured to control the instruction drive means (704) based on a transmission state of the drive force from the drive force transmission means (36); and
a control portion (37) configured to
determine whether or not the drive force transmission means (36) transmits the drive force from the bending power means (30) to the bending drive means (31, 32, 33, 34);
read out a current count value of the drive state detection means (30a) from the DPRAM (106) in the control portion (37) when it has been determined that the drive force transmission means (36) transmits the drive force from the bending power means (30) to the bending drive means (31, 32, 33, 34);
read out from the DPRAM (106) an output value of the bending state detection means (35), which has been obtained immediately after it has been determined that the drive force transmission means (36) does not transmit the drive force from the bending power means (30) to the bending drive means (31, 32, 33, 34);
read out a current output value of the bending state detection means (35);
calculate an amount of change, A, from the difference between the output value of the bending state detection means (35), which has been obtained immediately after it has been determined that the drive force transmission means (36) does not transmit the drive force from the bending power means (30) to the bending drive means (31, 32, 33, 34), and the current output value of the bending state detection means (35),
control the instruction drive control means (706) to drive the instruction drive means (704) on the side of the instruction means (701) based on the amount of change, A;
read in the position data of the potentiometer (702) on the side of the instruction means (701); and
update the current count value of the drive state detection means (30a) in the DPRAM (106) to a value corresponding to the position data of the potentiometer (702) on the side of the instruction means (701).

## Patentansprüche

1. Elektrisches Biegeendoskop (2) aufweisend:
ein Biegeteil (11), das in einem Einführungsteil (9) bereitgestellt ist;
Biegeantriebsmittel (31, 32, 33, 34) mit einer Mehrzahl von Komponenten, die dazu eingerichtet sind, das Biegeteil (11) zu biegen;
Biegeenergiemittel (30), die dazu eingerichtet sind, eine Antriebskraft zum Antreiben der Biegeantriebsmittel (31, 32, 33, 34) auszugeben;
eine Motorsteuerung (110), die dazu eingerichtet ist, die Biegeenergiemittel (30) zu steuern und Werte zu lesen von und zu schreiben in ein DPRAM (106);
Antriebskraftübertragungsmittel (36), die dazu eingerichtet sind, wahlweise die Antriebskraft von den Biegeenergiemitteln (30) an die Biegeantriebsmittel (31, 32, 33, 34) zu übertragen;
Antriebszustandserfassungsmittel (30a), die dazu eingerichtet sind, Information über einen Antriebszustand der Biegeenergiemittel (30) zu erfassen;
Biegezustandserfassungsmittel (35), die dazu eingerichtet sind, Betriebsinformation der Biegeantriebsmittel (31, 32, 33, 34) zu messen, um Information über einen Biegezustand des Biegeteils (11) zu erfassen;
Instruktionsmittel (701), die dazu eingerichtet sind,
Biegeinstruktionsinformation zum Biegen des Biegeteils (11) auszugeben;
ein Potentiometer (702), das dazu eingerichtet ist, Positionsdaten der Instruktionsmittel (701) zu erfassen;
Instruktionsantriebsmittel (704), die dazu eingerichtet sind, die Instruktionsmittel (701) anzutreiben;
Instruktionsantriebssteuerungsmittel (706), die dazu eingerichtet sind, die Instruktionsantriebsmittel (704) basierend auf einem Übertragungszustand der Antriebskraft von den Antriebskraftübertragungsmitteln (36) zu steuern; und
ein Steuerungsteil (37), das eingerichtet ist zum
Bestimmen, ob die Antriebskraftübertragungsmittel (36) die Antriebskraft von den Biegeenergiemitteln (30) zu den Biegeantriebsmitteln (31, 32, 33, 34) übertragen oder nicht;
Lesen eines gegenwärtigen Zählwerts der
Antriebszustandserfassungsmittel (30a) aus dem DPRAM (106) in dem Steuerungsteil (37), wenn bestimmt wurde, dass die Antriebskraftübertragungsmittel (36) die Antriebskraft von den Biegeenergiemitteln (30) zu den Biegeantriebsmitteln (31, 32, 33, 34) übertragen;
Lesen eines Ausgabewerts der Biegezustandserfassungsmittel (35), der unmittelbar erlangt wurde, nachdem bestimmt wurde, dass die
Antriebskraftübertragungsmittel (36) nicht die Antriebskraft von den Biegeenergiemitteln (30) an die Biegeantriebsmittel (31, 32, 33, 34) übertragen, aus dem DPRAM (106);
Auslesen eines gegenwärtigen Ausgabewerts der
Biegezustandserfassungsmittel (35);
Berechnen eines Änderungsbetrags, A, aus der Differenz zwischen dem Ausgabewert der Biebgezustandserfassungsmittel (35), der unmittelbar erlangt wurde, nachdem bestimmt wurde, dass die Antriebskraftübertragungsmittel (36) nicht die Antriebskraft von den Biegeenergiemitteln (30) an die Biegeantriebsmittel (31, 32, 33, 34) übertragen, und dem gegenwärtigen Ausgabewert der Biegezustandserfassungsmittel (35),
Steuern der Instruktionsantriebssteuerungsmittel (706) zum Antreiben der Instruktionsantriebsmittel (704) auf der Seite der Instruktionsmittel (701) basierend auf dem Änderungsbetrag, A;
Einlesen der Positionsdaten des Potentiometers (702) auf der Seite der Instruktionsmittel (701); und
Aktualisieren des gegenwärtigen Zählwerts der
Antriebszustandserfassungsmittel (30a) in dem DPRAM (106) auf einen Wert entsprechend den Positionsdaten des Potentiometers (702) auf der Seite der Instruktionsmittel (701).

## Revendications

1. Endoscope (2) à courbure électrique, comprenant :
une partie de courbure (11) prévue dans une partie d'insertion (9) ;
un moyen (31, 32, 33, 34) d'entraînement de courbure comportant une pluralité de composants configurés pour courber la partie de courbure (11) ;
un moyen (30) de puissance de courbure configuré pour délivrer en sortie une force d'entraînement pour entraîner le moyen (31, 32, 33, 34) d'entraînement de courbure ;
un contrôleur (110) de moteur configuré pour commander le moyen (30) de puissance de courbure et lire des valeurs à partir d'une RAM à double accès (106) et écrire des valeurs dans celle-ci ;
un moyen (36) de transmission de force d'entraînement configuré pour transmettre sélectivement la force d'entraînement du moyen (30) de puissance de courbure au moyen (31, 32, 33, 34) d'entraînement de courbure ;
un moyen (30a) de détection d'état d'entraînement configuré pour détecter des informations concernant un état d'entraînement du moyen (30) de puissance de courbure ;
un moyen (35) de détection d'état de courbure configuré pour détecter des informations de fonctionnement du moyen (31, 32, 33, 34) d'entraînement de courbure pour détecter des informations concernant un état de courbure de la partie de courbure (11) ;
un moyen (701) d'instruction configuré pour délivrer en sortie des informations d'instruction de courbure pour courber la partie de courbure (11) ;
un potentiomètre (702) configuré pour détecter des données de position du moyen (701) d'instruction ;
un moyen (704) d'entraînement d'instruction configuré pour entraîner le moyen (701) d'instruction ;
un moyen (706) de commande d'entraînement d'instruction configuré pour commander le moyen (704) d'entraînement d'instruction sur la base d'un état de transmission de la force d'entraînement depuis le moyen (36) de transmission de force d'entraînement ; et
une partie de commande (37) configurée pour
déterminer si oui ou non le moyen (36) de transmission de force d'entraînement transmet la force d'entraînement du moyen (30) de puissance de courbure au moyen (31, 32, 33, 34) d'entraînement de courbure ;
extraire une valeur de compte actuelle du moyen (30a) de détection d'état d'entraînement à partir de la RAM à double accès (106) dans la partie de commande (37) lorsqu'il a été déterminé que le moyen (36) de transmission de force d'entraînement transmet la force d'entraînement du moyen (30) de puissance de courbure au moyen (31, 32, 33, 34) d'entraînement de courbure ;
extraire à partir de la RAM à double accès (106) une valeur de sortie du moyen (35) de détection d'état de courbure, qui a été obtenue immédiatement après qu'il a été déterminé que le moyen (36) de transmission de force d'entraînement ne transmet pas la force d'entraînement du moyen (30) de puissance de courbure au moyen (31, 32, 33, 34) d'entraînement de courbure ;
extraire une valeur de sortie actuelle du moyen (35) de détection d'état de courbure ;
calculer une quantité de changement, A, à partir de la différence entre la valeur de sortie du moyen (35) de détection d'état de courbure, qui a été obtenue immédiatement après qu'il a été déterminé que le moyen (36) de transmission de force d'entraînement ne transmet pas la force d'entraînement du moyen (30) de puissance de courbure au moyen (31, 32, 33, 34) d'entraînement de courbure, et la valeur de sortie actuelle du moyen (35) de détection d'état de courbure,
commander le moyen (706) de commande d'entraînement d'instruction pour entraîner le moyen (74) d'entraînement d'instruction du côté du moyen (701) d'instruction sur la base de la quantité de changement, A ;
lire les données de position du potentiomètre (702) du côté du moyen (701) d'instruction ; et
mettre à jour la valeur de compte actuelle du moyen (30a) de détection d'état d'entraînement dans la RAM à double accès (106) à une valeur correspondant aux données de position du potentiomètre (702) du côté du moyen (401) d'instruction.
